# EUROPEAN PATENT APPLICATION

(11) **EP 4 607 189 A2**
(43) Date of publication of application: **27.08.2025**
(21) Application number: 25168229.0
(22) Date of filing: 04.06.2020
(51) Int. Cl.: G01N 30/72

(54) **UNIVERSAL LIPID QUANTITATIVE STANDARDS FOR USE IN LIPIDOMICS**

(30) Priority: 04.06.2019 US 201962857230 P
(62) Divisional of application: 20817919.2
(71) Applicant: Avanti Polar Lipids, LLC, Alabaster, Alabama 35007 (US)
(72) Inventor: BAKER, Paul RS, Alabaster, 35007 (US); CONNELL, Lisa, Alabaster, 35007 (US); SULLARDS, Cameron, Alabaster, 35007 (US); LI, Shengrong, Alabaster, 35007 (US)
(74) Representative: Best, Rachel Elizabeth

(57) **Abstract**

The present disclosure provides for a universal lipid quantitative standard (ULQS) comprising a plurality of isotopically labeled lipid standards that can be used with any analytical mass spectrometry techniques known in the art. The ULQS includes at least one isotopically labeled lipid species from one or more of the following lipid classes: i) phospholipids; ii) lysophospholipids; iii) cholesterol esters; iv) triacylglycerols; v) diacylglycerols; vi) ceramides; and vii) sphingomyelins.

## Description

### BACKGROUND

Lipids are naturally occurring organic molecules that can possess either both a hydrophobic and hydrophilic character (i.e., polar lipids such as phospholipids and sphingomyelins) or strictly hydrophobic character (i.e., neutral lipids such as tri-, di- and monoglycerides and sterol esters). Lipids are present in the cells of every living organism and play a complex role in a variety of physiological processes. In addition, specific lipid species are used as biomarkers for certain disease states (for example, coronary heart disease and infectious disease). Lipids are also present in foods and the monitoring of lipids can be used to verify the safety of food.

There is no single system or method for identifying and quantifying all the lipids present in a particular sample in a reasonable amount of time. This is due in part to the complexity of lipids as a class of compounds. The number of actual lipid substances within living cells is enormous. Nearly 40,000 unique structures of lipids have been catalogued in the LIPID MAPS database. Furthermore, the dynamic range in which lipid concentrations can vary in a biological samples may be 10¹² or more.

One aspect of lipid complexity is that families of closely related lipids are present in a sample that differ only by the number of fatty acyl carbons and/or the number/position of double bonds, as well as minor variantions in structure (for example, ether/ester substitution and double bond stereo configuration). These closely related families comprise molecular species that have very similar structure and molecular weights to one another. As such, many lipid species share the same mass (either in the precursor ion and/or the product ion). This phenomenon is referred to as isobaric interference, which greatly affects the analytical identification and quantitation of individual lipid molecular species.

Mass spectrometry has emerged as a powerful tool for the analysis of all lipids. Lipidomic analysis of biological systems using various approaches is now possible with a quantitative measurement of thousands of lipid molecular species in a single analytical run. Challenges still remain in the lipidomics area, in large part because it is very tedious to correctly develop a quantitative analysis method with the correct internal standard strategy. In quantitative MS, stable isotope-labeled analogs of the analyte(s) are used as internal standards to measure the ratio of signal intensities of the analyte and internal standard rather than any absolute intensity. The ratio is then converted to analyte concentration by a calibration curve generated using reference standards. However, this approach is not available for most lipidomic studies because of the absence of sufficient internal standards for a representative number of lipid species present in the sample. Furthermore, many internal standards for use in lipid analysis cannot correct for the issues of extraction efficiency, ionization efficiency, fragmentation efficiency (due to acyl chain length and the number of double bonds present in an acyl chain) and isobaric interference.

Therefore, the absence of internal standards reflective of the structural diversity (for example, number and position of double bonds in fatty acyl chains, variety of fatty acyl chain length, and positional isomers) and technical issues is a factor limiting the analysis of lipid by MS. As a result, there is a clear need for internal standards for use in the quantitative analysis of lipids by MS that address the issues inherent in the analysis of lipids by MS, such as, but not limited to, extraction efficiency, ionization efficiency, fragmentation efficiency due to acyl chain length, fragmentation efficiency due to the number of double bonds present in an acyl chain, and/or isobaric interference. Furthermore, the requisite internal standards must be able to be used equally well with the three general analytical MS strategies: normal phase chromatography, reverse-phase chromatography and infusion ("Shotgun") based methods of sample introduction into the mass spectrometer.

The present disclosure provides a solution to these issues and other unmet needs in the art regarding the analysis of lipids by MS by providing a universal lipid quantitative standard (ULQS) for use in the quantitation of lipids.

### BRIEF DESCRIPTION OF THE FIGURES

FIG. 1 shows an example of design principles for selecting lipid species for inclusion in the ULQS of the present disclosure.
FIG. 2A shows an example of an MS scan of PE and LPE species in a lipid sample.
FIG. 2B shows an example of the relationship (expressed in terms of total carbon count of the fatty acyl chains) between endogenous PE and LPE species in a lipid same and the PE and LPE species present in one embodiment of the ULQS of the present disclosure.
FIG. 2C shows an example of how endogenous lipid species in a sample are quantitated using one embodiment of the ULQS of the present disclosure.
FIG. 3 shows an exemplary distribution of ULQS SM and PC species as determined by HILIC MS.
FIG. 4 shows an exemplary calibration curve for ULQS PS species as determined by HILIC MS.

### DETAILED DESCRIPTION

### Definitions

All patent applications, patents, and printed publications which are cited to be incorporated by reference are incorporated herein by reference in the entireties (unless specifically noted), except for any definitions, subject matter disclaimers or disavowals, and except to the extent that the incorporated material is inconsistent with the express disclosure herein, in which case the language in this disclosure controls.

As used herein, an "isotopic label" produces a mass shift in the labeled molecule relative to the unlabeled molecule when analyzed by mass spectrometric techniques. Examples of suitable isotopic labels include deuterium (d or ²H), ¹³C, and ¹⁵N. The isotopic label can be incorporated at one or more positions in the molecule and one or more kinds of isotopic labels can be used on the same isotopically labeled molecule. In a preferred embodiment, the isotopic labels are the same. In another preferred embodiment, the isotopic labels are the same and the isotopic label is deuterium.

In the present specification, lipids are referred to according to the following nomenclature: CE is cholesteryl ester, CER is ceramide, SM is sphingomyelin, TAG is triacylglycerol, DAG is diacylglycerol, PL is phospholipid, PC is phosphatidylcholine, PE is phosphatidylethanolamine, PS is phosphatidylserine, PG is phosphatidylglycerol, PI is phosphatidylinositol, LPL is lysophospholipid, LPC is lysophosphatidylcholine, LPE is lysophosphatidylethanolamine, LPS is lysophosphatidylserine, LPG is lysophosphatidylglycerol, and LPI is lysophosphatidylinositol.

The nomenclature X:Y indicates, X number of total carbon atoms in the fatty acid(s) portions of the molecule, and Y the total number of double bonds in the fatty acid portion(s) of the molecule. For example, CE 16:1 represents a cholesteryl ester with an acyl chain of 16 carbon atoms containing a single double bond. Multiple X:Y designations may be used when more than on acyl chain is present. For example, TAG 16:0/15:1/16:0 represents a triacylglycerol molecule with 3 acyl chains, with two saturated acyl chains being 16 carbon atoms in length and one unsaturated acyl chain being 15 carbon atoms in length with a single double bond.

As used herein the term "substantially" when referencing a particular quantity or range means at least 80% or greater of such quantity or range, such as 90% or greater, 95% or greater, or 98% or greater.

As used herein the term "saturated" with respect to an acyl chain means the acyl chain does not contain a double or triple bond.

As used herein the term "sum composition score" or "sum composition" refers to the combination of the sum of carbon atoms and the sum of double bonds on all fatty acyl moieties in a lipid species (for example, CE 16:1 representing a cholesteryl ester with an acyl chain of 16 carbon atoms containing a single double bond or PE 39:4 which could represent a PE with one acyl chain of 17 carbon atoms and no double bonds and one acyl chain of 22 carbon atoms and 4 double bonds). A sum composition score may refer individually to the acyl chain portion of the double bond portion.

As used herein the term "unsaturated" with respect to an acyl chain means the acyl chain contains a double or triple bond, preferably a double bond.

Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

### Overview of Mass Spectrometry Techniques

The described ULQS is designed for use with samples to be analyzed by mass spectrometry (MS). MS is performed using a mass spectrometer, which includes an ionization source for ionizing the sample (which may be fractionated prior to being introduced into the mass spectrometer) and creating charged molecules for further analysis. For example, ionization of the lipid species in the sample may be performed by electron ionization, chemical ionization, electrospray ionization (ESI), photon ionization, atmospheric pressure chemical ionization (APCI), photoionization, atmospheric pressure photoionization (APPI), laser diode thermal desorption (LDTD), fast atom bombardment (FAB), liquid secondary ionization (LSI), matrix assisted laser desorption ionization (MALDI), field ionization, field desorption, thermospray/plasmaspray ionization, surface enhanced laser desorption ionization (SELDI), inductively coupled plasma (ICP) and particle beam ionization. In preferred embodiments, the sample are ionized by ESI.

Samples may be introduced into a mass spectrometer directly (for example, in Shotgun-based methods) or may be introduced into a mass spectrometer after the components of the sample have been fractionated (for example, in normal phase chromatography or reverse phase chromatography techniques).

MS techniques may be conducted in positive or negative ionization mode. In MS techniques generally, after the sample has been ionized, the positively or negatively charged ions created thereby may be analyzed to determine a mass-to-charge ratio. Suitable analyzers for determining mass-to-charge ratios include quadrupole analyzers, ion traps analyzers, and time-of-flight analyzers. A MS system may detect ions using several detection modes. For example, selected ions may be detected using a selective ion monitoring mode (SIM). In certain embodiments, the mass-to-charge ratio is determined using a quadrupole analyzer. For example, in a "quadrupole" or "quadrupole ion trap" instrument, ions in an oscillating radio frequency field experience a force proportional to the DC potential applied between electrodes, the amplitude of the RF signal, and the mass/charge ratio. The voltage and amplitude may be selected so that only ions having a particular mass/charge ratio travel the length of the quadrupole, while all other ions are deflected. Thus, quadrupole instruments may act as both a "mass filter" and as a "mass detector" for the ions injected into the instrument.

In another embodiment, the resolution of the MS technique may be improved by employing "tandem mass spectrometry," also referred to as "MS/MS," which is used to monitor mass transitions resulting from collision induced dissociation or neutral loss and may be monitored using, for example, multiple reaction monitoring (MRM) or selected reaction monitoring (SRM). In MS/MS, a precursor ion (also called a parent ion) generated from a lipid of interest can be filtered in the MS instrument, and the precursor ion subsequently fragmented to yield one or more fragment ions (also called product ions) that are then analyzed in a second MS procedure. The generated ions pass through the orifice of the instrument and enter a series of three quadrupoles (Q1, Q2, and Q3). Q1 acts as a mass filter, allowing selection of ions (i.e., selection of "precursor" ions) to pass into Q2 based on their mass to charge ratio (m/z). Q2 acts as a collision chamber where precursor ions are fragmented into fragment ions. Q3 acts as a mass filter allowing for selection of ions (i.e. fragment ions) based on their m/z. The three quadrupoles select for ions with the mass to charge ratios of fatty acids ions of interest. Ions with the correct mass/charge ratios are allowed to pass the quadrupoles and collide with the detector. By selection of precursor ions, only ions produced by certain analytes are passed to the fragmentation chamber, where collisions with atoms of an inert gas produce the fragment ions. Because both the precursor and fragment ions are produced in a reproducible fashion under a given set of ionization/fragmentation conditions, the MS/MS MRM scan mode provides for reliable and the most sensitive analytical results. Alternate modes of operating a tandem mass spectrometric instrument include precursor ion and neutral loss scanning modes. Such methods are known to those of skill in the art.

As ions collide with the detector they produce a pulse of electrons that is converted to a digital signal via an electron multiplier. The acquired data signal is relayed to a computer, which plots counts of the ions collected versus time. The areas under the peaks corresponding to particular ions, or the amplitude of such peaks, may be measured and correlated to the amount of the analyte of interest. As described above, the relative abundance of a given ion may be converted into an accurate quantitation of the original analyte using the ULQS as an internal standard.

### Quantitation of Lipids

There are many methods used to quantify lipids in a sample using MS. Generally, the methods for quantitation are classified as: i) absolute quantification; ii) relative quantification; and iii) accurate quantification.

Absolute quantification is based on having an internal standard and primary reference standard for every lipid molecule of interest (the lipid to be quantitated) in the sample. This approach is not possible for lipidomics due to the number of lipids and variety of lipids present in the sample. Relative quantification is based on using a single internal standard (for example, for use with infusion and HILIC-based analytical methods) per class of lipid to be analyzed. The molecular species within each lipid class are reported as area ratios relative to the single internal standard. Using this approach, the relative amount of lipid species between two different samples may be compared (noting that relative quantification does not provide a quantity of the lipid species).

In contrast to the above, accurate quantification is based on using multiple internal standards per lipid class in order to determine the amount of a lipid species present in the sample. Accurate quantitation at either the sum composition/score level or molecular species level may be obtained using the ULQS as described herein with a variety of MS techniques, including but not limited to, hydrophilic interaction liquid chromatography (HILIC), reverse-phase, and shotgun based techniques.

### Universal Lipid Quantitative Standards

The present disclosure provides for a universal ULQS comprising a plurality of isotopically labeled lipid internal standards. The ULQS can be used with any analytical mass spectrometry techniques known in the art, including those described herein.

In a general embodiment, the ULQS includes at least one isotopically labeled lipid species from one or more of the following lipid classes: i) a phospholipid class; ii) a lysophospholipid class; iii) a cholesterol ester class; iv) a triacylglycerol class; v) a diacylglycerol class; vi) a ceramide class; and vii) a sphingomyelin class.

Unless otherwise specifically stated herein, reference to a lipid or a lipid species included in the ULQS is understood to the lipid or lipid species is isotopically labelled as described herein. In a specific embodiment of the foregoing, the ULQS contains more than 1 lipid species from one or more of the seven classes described above. The ULQS may contain 2, 3, 4, or 5 or more different lipid species from one or more of the seven classes described above. The upper limit of lipid species from a given class is generally less than or equal to 100, such as less than or equal to 50 or less than or equal to 25.

In one embodiment, the ULQS contains 3 to 50 different lipid species from one or more of the seven classes described above. For clarity, the number of lipid species from each lipid class are not required to be the same. For example, with reference to the seven lipid classes described above, an exemplary ULQS may contain 15 phospholipid species, 10 lysophospholipid species, 3 cholesterol ester species, 5 triacylglycerol species, 3 diacylglycerol species, 5 ceramide species, and 5 sphingomyelin species. As another example, with reference to the seven lipid classes described above, an exemplary ULQS may contain 20 phospholipid species, 15 lysophospholipid species, 5 triacylglycerol species, 5 diacylglycerol species, and 5 sphingomyelin species. As a further example, with reference to the seven lipid classes described above, an exemplary ULQS may contain 25 phospholipid species, 15 lysophospholipid species, 3 cholesterol ester species, 9 triacylglycerol species, 5 diacylglycerol species, 5 ceramide species, and 5 sphingomyelin species.

The composition of the ULQS may be influenced by a variety of factors, including, but not limited to, the nature of the sample matrix (for example, plasma or urine), the source of the sample matrix (for example, human or mouse), and the identity of the lipid species to be analyzed. Furthermore, when the ULQS is used in methods to determine a lipid species associated with a disease, the nature of the disease may also influence the composition of the ULQS.

In selecting the lipid classes and the various lipid species from each class for inclusion in the ULQS, the lipid species are selected to correct for, among other things, at least one of the following: ionization efficiency, extraction efficiency, and differential fragmentation efficiency of at least one lipid species in the sample to be analyzed or tested. In certain embodiments, the various lipid species from each class for inclusion in the ULQS are selected to correct for differential fragmentation efficiency and at least one of the following: ionization efficiency and extraction efficiency. In certain embodiments, the various lipid species from each class for inclusion in the ULQS are selected to correct for differential fragmentation efficiency and both ionization efficiency and extraction efficiency. In any of the foregoing embodiments, the various lipid species from each class for inclusion in the ULQS are further selected to cover the entire mass range or substantially the entire mass range of at least one lipid species present in the sample. In any of the foregoing embodiments, the concentration of the various lipid species from each class of lipids selected for inclusion in the ULQS are selected to mirror the concentration of at least one lipid species present in the sample. In any of the foregoing embodiments, the various lipid species from each class for inclusion in the ULQS are further selected to cover the entire mass range of at least one lipid species present in the sample and the concentration of at least one lipid species are selected to mirror the concentration of lipid species present in the sample.

Generally, the concentration of the lipid species from each class included in the ULQS is from 1 to 1,000 µg/ml, preferably from 1 to 500 µg/ml. The concentration of lipid species between lipid classes may be the same or may be different. The concentration of lipid species within a lipid classes may be the same or may be different. In one embodiment, the concentration of the lipid species from the phospholipid and triacylglycerol classes included in the ULQS is from 1 to 1,000 µg/ml, preferably from 1 to 500 µg/ml, more preferably from 10 to 250 µg/ml, and even more preferably from 15 to 200 µg/ml. In one embodiment, the concentration of the lipid species from the lysophospholipid class included in the ULQS is from 1 to 500 µg/ml, preferably from 10 to 200 µg/ml, more preferably from 15 to 150 µg/ml, and even more preferably from 20 to 100 µg/ml. In one embodiment, the concentration of the lipid species from the ceramide, diacylglycerol, sphingomyelin, and cholesterol ester classes included in the ULQS is from 1 to 500 µg/ml, preferably from 10 to 350 µg/ml, more preferably from 15 to 250 µg/ml, and even more preferably from 20 to 150 µg/ml. In one embodiment, the concentration of the lipid species from the phospholipid, triacylglycerol, lysophospholipid, ceramide, diacylglycerol, sphingomyelin, and cholesterol ester classes is as described in Table 8 herein.

In certain embodiments, the ionization efficiency, extraction efficiency, and differential fragmentation efficiency are influenced by the nature of the sample matrix, the source of the sample matrix, and the identity of the lipid species to be analyzed.

For a typical human sample appropriate mass ranges (sum composition scores) for the various lipid classes described above are: i) phospholipids- 27:1 to 43:4 (preferably 31:1 to 39:4); ii) lysophospholipids- 11:0 to 23:0 (preferably 15:0 to 19:0); iii) cholesterol esters- 11:1 to 25:4 (preferably 14:1 to 22:4); iv) triacylglycerols- 43:1 to 55:2 (preferably 47:1 to 51:2); v) diacylglycerols- 27:1 to 43:4 (preferably 31:1 to 39:4); vi) ceramides- 12:1 to 28:1 (preferably 16:1 to 24:1); and vii) sphingomyelins- 12:1 to 28:1 (preferably 16:1 to 24:1).

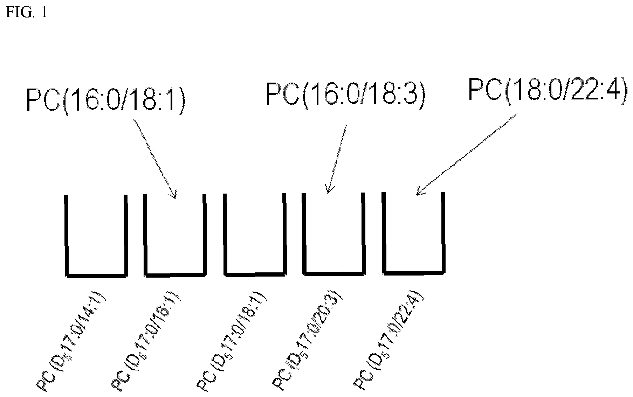

The ULQS internal strategy design approach may be visualized as shown in FIG. 1. Each lipid species of the ULQS for a given lipid class defines a quantification window (or bucket). Each lipid species in the sample is assigned to a particular quantification window for quantification. If there is not an exact match, the best approximation is used. In FIG. 1 the ULQS contains 5 lipid species for the PC class designated as PC(D₅17:0/14:1), PC(D₅17:0/16:1), PC(D₅17:0/18:1), PC(D₅17:0/20:3), and PC(D₅17:0/22:4). The 5 PC species define the quantification windows to which the lipid species in the sample are assigned. The three lipid species in the sample are shown for illustrative purposes as PC(16:0/18:1), PC(16:0/18:3), and PC(18:0/22:4). None of the lipid species in the sample is an exact match for the defined quantification windows defined by the ULQS lipid species so the best approximation is used. In this example, the PC(16:0/18:1) lipid in the sample is assigned to the quantification window defined by the PC(Ds17:0/16:1) ULQS species, the PC(16:0/18:3) lipid in the sample is assigned to the quantification window defined by the PC(D₅17:0/20:3) ULQS species, and the PC(16:0/22:4) lipid in the sample is assigned to the quantification window defined by the PC(D₅17:0/22:4) ULQS species.

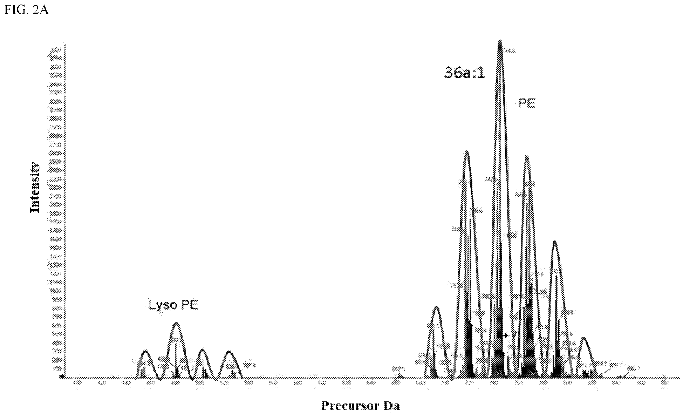

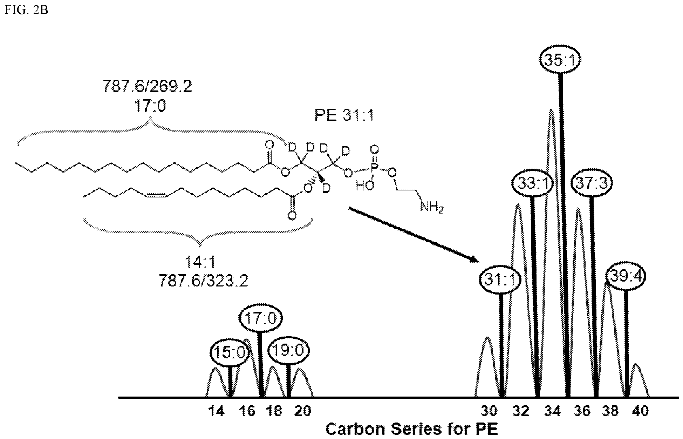

An exemplary design strategy for the design of a ULQS is shown in FIGS. 2A to 2C, illustrating the design strategy with respect to LPE and PE. The same approach may be used to design components of the ULQS for other lipid species. In FIG. 2A, a MS scan (intensity vs Precursor ion Da) of an exemplary lipid sample is shown illustrating various LPE and PE species present in the sample. The outline over the various species indicates the location and relative abundance of each LPE and PE species in the particular sample. FIG. 2B shows the same MS scan shown in FIG. 2A modified to show the presence of exemplary ULQS LPE and PE species (i.e., internal standards) relative to the LPE and PE species in terms of the sum composition score. In this example, the ULQS LPE species are LPE 15:0, LPE 17:0, and LPE 19:0, with sum composition scores of 15:0, 17:0, and 19:0, respectively, and the ULQS PE species are PE(D₅17:0/14:1), PE(D₅17:0/16:1), PE(D₅17:0/18:1), PE(D₅17:0/20:3), and PE(D₅17:0/22:4), with sum composition scores of 31:1, 33:1, 35:1, 37:3, and 39:4, respectively. FIG. 2B also shows the structure of the ULQS PE 31:1 standard indicating the MRM transitions for the 17:0 fatty acyl moiety (787.6/269.3) and the 14:1 fatty acyl moiety (787.6/323.3). MRM transitions for the any lipid species present in the ULQS may be determined by the person of ordinary skill in the art. Based on the molecular weight of the deuterated species, minimal isobaric interference with endogenous lipid species is expected. In this example, the PE class has 5 species and the LPE class has three species (although as discussed herein, the number of species for each class may be increased or decreased) with the selection of the PE and LPE species selected to account for ionization efficiency, extraction efficiency, and differential fragmentation efficiency of the PE and LPE lipid species in the sample and to cover substantially the entire mass range of the PE and LPE lipid species in the sample. In addition, the incorporation of odd chain fatty acids in the PE and LPE internal standards minimizes isobaric overlap and improves MRM specificity. Finally, the concentration of each internal standard present in the ULQS is selected to reflect the relative abundance of the endogenous lipid species within each class present in the sample.

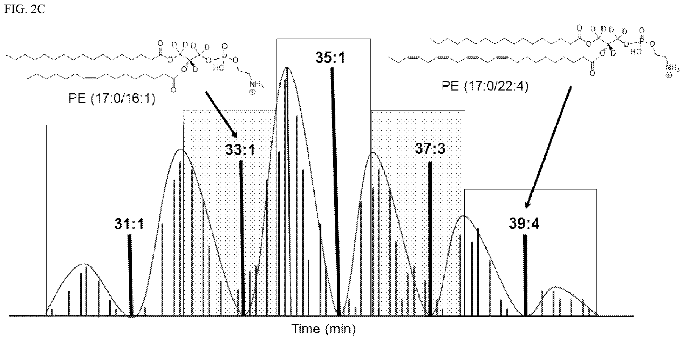

FIG. 2C illustrates how the ULQS species are used to quantitate the various PE lipid species present in the sample. As described above, each ULQS PE species define quantitation windows and the various PE lipid species in the sample are assigned to a quantitation window based on an exact match or best approximation. In this example, the quantitation window for each ULQS PE species is shown in the alternating unshaded and shaded boxes and the various PE lipid species in the sample falling within the various boxes are quantitated using the designated ULQS PE quantitation window. The same approach may be used to quantitate the LPE lipid species (as well as any other lipid species present in the sample in the presence of appropriate ULQS internal standards).

Having internal standards distributed across the range of masses for each class allows normalization for ionization efficiency, extraction efficiency, and differential fragmentation efficiency of lipid species in the sample based on fatty acid/molecular weight. In addition, as hydrophobicity of the lipid species (of both the ULQS species and the lipid species present in the sample) is roughly proportional to molecular weight/fatty acid chain lengths, the ULQS internal standards selected as described herein will distribute along a gradient (for example in reverse-phase analysis) in the same manner as the lipid species in the sample, thereby improving quantitative accuracy.

The resulting ULQS may be used in the accurate quantitation of lipid species in a sample. Accurate quantitation is important for a variety of reasons. First, the accurate quantification of lipids in a sample provides for standardization of results, allowing the data generated between different labs to be compared and allowing the comparison of the concentration of different lipid species within a sample to be compared. Such standardization will improve the ability of investigators to collaborate and share data. In addition, accurate quantitation has clinical applications, allowing for the establishment of reference values for various lipid species and allowing for the use of lipid species as biomarkers in diagnostic applications.

In one embodiment, the lipid species for inclusion in the ULQS include 3 to 50 different lipid species from one or more of the seven classes described above. In certain embodiments, an exemplary ULQS may contain: i) 15 phospholipid species, 10 lysophospholipid species, 3 cholesterol ester species, 5 triacylglycerol species, 3 diacylglycerol species, 3 ceramide species, and 3 sphingomyelin species; ii) 20 phospholipid species, 15 lysophospholipid species, 5 cholesterol ester species, 9 triacylglycerol species, 5 diacylglycerol species, 5 ceramide species, and 5 sphingomyelin species; or iii) 25 phospholipid species, 15 lysophospholipid species, 5 cholesterol ester species, 9 triacylglycerol species, 5 diacylglycerol species, 5 ceramide species, and 5 sphingomyelin species. Regardless of the composition of the ULQS, the individual lipid species selected for inclusion in the ULQS are selected to correct for, among other things, at least one of the following: ionization efficiency, extraction efficiency, and differential fragmentation efficiency of at least one lipid species in the sample to be analyzed/tested. In certain embodiments, the individual lipid species selected for inclusion are selected to correct for differential fragmentation efficiency and at least one of ionization efficiency and extraction efficiency (preferably both ionization efficiency and extraction efficiency). In certain embodiments, the individual lipid species selected for inclusion in the ULQS are selected to correct for at least one of the following: ionization efficiency, extraction efficiency, and differential fragmentation efficiency of at least one lipid species in the sample to be tested and the various lipid species for inclusion in the ULQS from each class are further selected to: i) cover the entire mass range or substantially the entire mass range of at least one lipid species present in the sample; ii) to mirror the concentration of at least one lipid species present in the sample; or iii) cover the entire mass range or substantially the entire mass range of at least one lipid species present in the sample and to mirror the concentration of at least one lipid species present in the sample.

In certain embodiments, the individual lipid species selected for inclusion in the ULQS are selected to correct for differential fragmentation efficiency of at least one lipid species in the sample to be tested and at least one of ionization efficiency and extraction efficiency (preferably both ionization efficiency and extraction efficiency) of at least one lipid species in the sample to be analyzed/tested and the various lipid species for inclusion in the ULQS from each class are further selected to: i) cover the entire mass range or substantially the entire mass range of at least one lipid species present in the sample; ii) to mirror the concentration of at least one lipid species present in the sample; or iii) cover the entire mass range or substantially the entire mass range of at least one lipid species present in the sample and to mirror the concentration of at least one lipid species present in the sample.

### Phospholipids

In one embodiment, the phospholipid class includes a plurality of phospholipid species, wherein each phospholipid species contains an isotopic label. Any combination of phospholipid species described herein may be used in the ULQS. In one embodiment, the phospholipid class includes one or more phospholipid species selected from the group consisting of: phosphatidylcholine (PC), phosphatidylethanolamine (PE), phosphatidylserine (PS), phosphatidylglycerol (PG), and phosphatidylinositol (PI). In each of the foregoing, the acyl chains of the phospholipid species are independently selected from saturated C3 to C30 chains or unsaturated C3 to C30 acyl chains, containing from 1 to 6 double bonds, such as from 1 to 2 or 1 to 4 double bonds.

In one embodiment, the acyl chains of the phospholipid species are each independently a saturated or unsaturated C3 to C6 acyl chain, a saturated or unsaturated C7 to C11 acyl chain, a saturated or unsaturated C12 to C21 acyl chain, a saturated or unsaturated C22 to C30 acyl chain. Such acyl chains, regardless of the length, includes both even and odd chain lengths and may be saturated or unsaturated. In a particular embodiment, the acyl chain length of the phospholipid species are each selected such that the sum composition score for the acyl chains is an odd number. In a particular embodiment, the acyl chains of the phospholipid species are each independently an acyl chain of 3, 4, 5, 6, 7, 8, 9, 10, 11, 12, 13, 14, 15, 16, 17, 18, 19, 20, 21, 22, 23, 24, 25, 26, 27, 28, 29, or 30 carbons, which are saturated or unsaturated. In another particular embodiment, the acyl chains of the phospholipid species are each independently an acyl chain of 10, 11, 12, 13, 14, 15, 16, 17, 18, 19, 20, 21, 22, 23, 24 or 25 carbons, which are saturated or unsaturated. In still another particular embodiment, the acyl chains of the phospholipid species are each independently an acyl chain of 15, 16, 17, 18, 19, 20, 21, 22, or 23 carbons, which are saturated or unsaturated.

In one embodiment, one or both of the acyl chains of the phospholipid species are saturated. In another embodiment, one or both of the acyl chains of the phospholipid species are unsaturated. In still another embodiment, one of the acyl chains is saturated and one of the acyl chains is unsaturated. When one or both of the acyl chains of the phospholipid species are unsaturated, the acyl chain may contain from 1 to 6, from 1 to 4 or from 1 to 3 double bonds. In one embodiment, at least one of the acyl chains are unsaturated, containing from 1 to 4, from 1 to 3 or from 1 to 2 double bonds. In certain embodiments, at least one of the acyl chains contains 2 or more double bonds when the acyl chain length is over 18 or over 20 (containing from example from 2 to 4 double bonds). The double bonds in such unsaturated acyl chains may be present in the cis or trans configuration, or a mixture of cis and trans configuration when more than 1 double bond is present.

In one embodiment, when multiple species of a particular phospholipid (for example, multiple phosphatidylcholine species) are present, the acyl chains on the various species are selected to include both saturated and unsaturated acyl chains (for example, with 1 to 4 double bonds). In one embodiment, when multiple species of a phospholipid are present, the acyl chains on the various species are selected to include both saturated and unsaturated acyl chains (for example, with 1 to 4 double bonds) and includes at least one acyl chain with 2 to 4 double bonds.

In certain embodiments, when multiple species of a particular phospholipid (for example, multiple phosphatidylcholine species) are present, the length of the acyl chains of the different species are different from one another. In certain embodiments, when multiple species of a particular phospholipid (for example, multiple phosphatidylcholine species) are present, the length of the acyl chains of the different species are different from one another and one of the acyl chains is unsaturated (containing for example 1 to 4 double bonds) and the other acyl chain is saturated (contains no double bonds).

In any of the foregoing embodiments, each phospholipid species contains at least one isotopically labeled H atom, preferably from 2 to 5 isotopically labeled H atoms. In preferred embodiments, the isotopically labeled H atom is deuterium (D).

In one embodiment of the foregoing, the phospholipid species for inclusion in the ULQS are represented by the general formula I, or a pharmaceutically acceptable salt thereof: 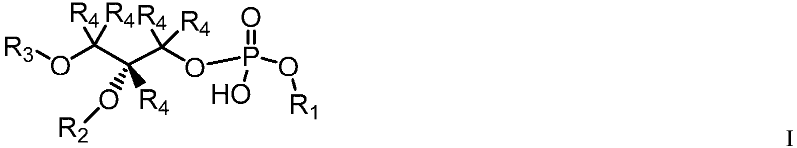 wherein:
R₁ is a head group moiety;
R₂ is a C3 to C30 saturated or unsaturated acyl chain;
R₃ is a C3 to C30 saturated or unsaturated acyl chain; and
R₄ is independently H or an isotope of H, provided that at least one of R₄ is an isotope of H.

R₁ may be selected from any phospholipid head group known in the art. Suitable selections for R₁ include, but are not limited to, -(CH₂)ₙ-N(CH₃)₃, -(CH₂)ₙ-NH₃, -(CH₂)ₙ-C(H)(NH3)-C(O)-O-, -(CH₂)ₙ-CH(OH)-CH(OH), inositol, and H, where n is independently selected from 1 to 6, preferably 1 to 2.
Particularly preferred substituents for R₁ are of the formula (A), (B), (C), (D), and (E) below: 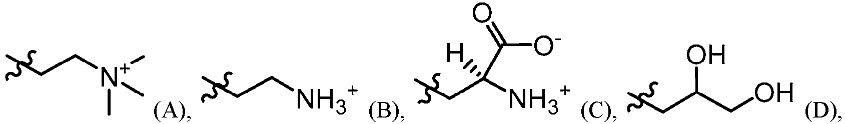 and 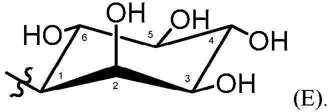 When R₁ is (A), the phospholipid is phosphatidylcholine (PC). When R₁ is (B), the phospholipid is phosphatidylethanolamine (PE). When R₁ is (C), the phospholipid is phosphatidylserine (PS). When R₁ is (D), the phospholipid is phosphatidylglycerol (PG). When R₁ is (E), the phospholipid is phosphatidylinositol (PI).

The descriptions below for R₂, R₃, and R₄ are applicable to phospholipid species containing any head group described herein.

R₂ is a saturated or unsaturated acyl chain from 3 to 30 carbons in length. In one embodiment, R₂ is an acyl chain from 3 to 6 carbons in length, an acyl chain from 7 to 11 carbons in length, an acyl chain from 12 to 21 carbons in length, or an acyl chain from 22 to 30 carbons in length. In a particular aspect, R₂ is an acyl chain from 10 to 25 carbons in length. In another particular aspect, R₂ is an acyl chain from 14 to 22 carbons in length. Such acyl chain, regardless of the length, includes both even and odd chain lengths and may be saturated or unsaturated. In a particular embodiment, R₂ is an acyl chain having an even number of carbon atoms In another embodiment, R₂ is an acyl chain of 3, 4, 5, 6, 7, 8, 9, 10, 11, 12, 13, 14, 15, 16, 17, 18, 19, 20, 21, 22, 23, 24, 25, 26, 27, 28, 29 or 30 carbons, which are saturated or unsaturated. In another embodiment, R₂ is an acyl chain of 10, 11, 12, 13, 14, 15, 16, 17, 18, 19, 20, 21, 22, 23, 24 or 25 carbons, which is saturated or unsaturated. In another embodiment, R₂ is an acyl chain of 14, 15, 16, 17, 18, 19, 20, 21, or 22 carbons, which are saturated or unsaturated.

In one embodiment, R₂ is a saturated acyl chain. In another embodiment, R₂ is an unsaturated acyl chain. When R₂ is an unsaturated acyl chain, the acyl chain may contain from 1 to 6, from 1 to 4 or from 1 to 3 double bonds. In one embodiment, R₂ is an unsaturated acyl chain; containing from 1 to 4, from 1 to 3 or from 1 to 2 double bonds. In certain embodiments, R₂ is contains 2 or more double bonds when the acyl chain length is over 18 or over 20 (containing from example from 2 to 4 double bonds). The double bonds in such unsaturated acyl chains may be present in the cis or trans configuration, or a mixture of cis and trans configuration when more than 1 double bond is present.

In one embodiment, when multiple species of a particular phospholipid (for example, multiple phosphatidylcholine species) are present, the R₂ groups on the various species are selected to include both saturated and unsaturated acyl chains (for example, with 1 to 4 double bonds). In one embodiment, when multiple species of a phospholipid are present, the R₂ groups on the various species are selected to include only unsaturated acyl chains (for example, with 1 to 4 double bonds). In one embodiment, when multiple species of a phospholipid are present, the R₂ groups on the various species are selected to include only unsaturated acyl chains (for example, with 1 to 4 double bonds) and includes acyl chains with 2 to 4 double bonds when the acyl chain length is over 18 or over 20.

In certain embodiments, the length of the acyl chain of R₂ is different from the length of the acyl chain of R₃. In certain embodiments, the length of the acyl chain of R₂ is different from the length of the acyl chain of R₃ and R₂ is unsaturated (containing for example 1 to 4 double bonds, particularly 2 to 4 double bonds when the acyl chain length is over 18 or over 20) and R₃ is saturated (contains no double bonds).

R₃ is a saturated or unsaturated acyl chain from 3 to 30 carbons in length. In one embodiment, R₃ is an acyl chain from 3 to 5 carbons in length, an acyl chain from 6 to 10 carbons in length, an acyl chain from 11 to 20 carbons in length, or an acyl chain from 21 to 30 carbons in length. In a particular aspect, R₃ is an acyl chain from 10 to 20 carbons in length. In another particular aspect, R₃ is an acyl chain from 15 to 20 carbons in length. Such acyl chain, regardless of the length, includes both even and odd chain lengths and may be saturated or unsaturated. In a particular embodiment, R₃ is an acyl chain having an odd number of carbon atoms. In one embodiment, the acyl chain is saturated. In another embodiment, R₃ is an acyl chain of 10, 11, 12, 13, 14, 15, 16, 17, 18, 19, 20, 21, 22, 23, 24, 25, 26, 27, 28, 29 or 30 carbons, which are saturated or unsaturated. In another embodiment, R₃ is an acyl chain of 10, 11, 12, 13, 14, 15, 16, 17, 18, 19, or 20 carbons, which is saturated or unsaturated. In another embodiment, R₃ is an acyl chain of 15, 16, 17, 18, 19, or 20 carbons, which are saturated or unsaturated.

In one embodiment, R₃ is a saturated acyl chain. In another embodiment, R₃ is an unsaturated acyl chain. When R₃ is an unsaturated acyl chain (regardless of length), the acyl chain may contain from 1 to 4 or from 1 to 3 double bonds. In one embodiment, R₃ is an unsaturated acyl chain, containing 1 or 2 double bonds. The double bonds in such unsaturated acyl chains may be present in the cis or trans configuration, or a mixture of cis and trans configuration when more than 1 double bond is present.

In one embodiment, when multiple species of a phospholipid are present, the R₃ groups on the various species are selected to include only saturated acyl chains.

In certain embodiments, the length of the acyl chain of R₃ is different from the length of the acyl chain of R₂. In certain embodiments, the length of the acyl chain of R₃ is different from the length of the acyl chain of R₂ and R₃ is saturated (contains no double bonds) and R₂ is unsaturated (containing for example 1 to 4 double bonds, particularly 2 to 4 double bonds when the acyl chain length is over 18 or over 20).

In one embodiment of any of the foregoing, the isotopically labeled H is deuterium (D). In one embodiment of any of the foregoing, at least 1, at least 2, at least 3, at least 4, or all 5 of R₄ are isotopically labeled H, preferably deuterium (D).

In certain embodiments, at least a subset of the phospholipid species included in the ULQS have the same R₃ group, but different R₂ groups. In certain embodiments, at least a subset of the phospholipid species included in the ULQS have the same R₃ group, but different R₂ groups, wherein at least one or all of the R₂ groups are unsaturated (for example, containing 1 to 4 double bonds, particularly 2 to 4 double bonds when the acyl chain length is over 18 or over 20).

In certain embodiments, the phospholipid species included in the ULQS have each of the head groups of formula (A), (B), (C), (D), and (E). In certain embodiments, the phospholipid species included in the ULQS have each of the head groups of formula (A), (B), (C), (D), and (E), the phospholipid species have the same R₃ group, but different R₂ groups. In certain embodiments, the phospholipid species included in the ULQS have each of the head groups of formula (A), (B), (C), (D), and (E) the phospholipid species have the same R₃ group, but different R₂ groups wherein at least one or all of the R₂ groups are unsaturated (for example, containing 1 to 4 double bonds, particularly 2 to 4 double bonds when the acyl chain length is over 18 or over 20).

In certain embodiments, the phospholipid species included in the ULQS are at least one each of PC, PE, PS, PG, and PI, the phospholipid species have the same R₃ group, but different R₂ groups wherein the length of the R₃ group is n and the length of the R₂ group is from n-5 to n+7, and at least one or all, preferably all, of the R₂ groups are unsaturated (for example, containing 1 to 4 double bonds), particularly 2 to 4 double bonds when the acyl chain length is over 18 or over 20). In one aspect of this embodiment, n is 12 to 21.

In one embodiment, phospholipid species included in the ULQS are at least one each of PC, PE, PS, PG, and PI, at least one of R₄ (preferably all of R₄) is deuterium (D), R₃ is a saturated acyl chain of n carbons in length, R₂ is an unsaturated acyl chain of n-5 to n+7 carbons in length, and n is 12 to 21, wherein R₂ contains 1 or 2 double bonds (preferably 1 double bond) when the length of R₂ is from n-5 to n+1, R₂ contains 2 or 3 double bonds (preferably 3 double bonds) when the length of R₂ is from n+2 to n+4, and R₂ contains 3 or 4 double bonds (preferably 4 double bonds) when the length of R₂ is from n+5 to n+7. In one aspect of this embodiment, 3-5 species are present for each of PC, PE, PS, PG, and PI and each of the 15-25 species are different. In another aspect of this embodiment, 3-5 species are present for each of the PC, PE, PS, PG, and PI species, each of the 15-25 species are different, and the R₃ and R₂ groups are repeated for each of the PC, PE, PS, PG, and PI species. For example, if 5 PC species have saturated acyl chains of 16 carbons in length for R₃ and unsaturated acyl chains of 13, 15, 17, 19, and 21 carbons in length for R₂, then the 5 PE, PS, PG, and PI species will also have saturated acyl chains of 16 carbons for R₃ and unsaturated (with the same number of double and/or triple bonds at the same position(s)) acyl chains of 13, 15, 17, 19, and 21 carbons for R₂.

In one embodiment, phospholipid species included in the ULQS are PC, PE, PS, PG, and PI, at least one of R₄ (preferably all of R₄) is deuterium (D), R₃ is a saturated acyl chain of n carbons in length, R₂ is an unsaturated acyl chain of n-5 to n+7 carbons in length, and n is 13, 15, 17, 19, or 21, R₂ contains 1 or 2 double bonds (preferably 1 double bond) when the length of R₂ is from n-5 to n+1, R₂ contains 2 or 3 double bonds (preferably 3 double bonds) when the length of R₂ is from n+2 to n+4, and R₂ contains 3 or 4 double bonds (preferably 4 double bonds) when the length of R₂ is from n+5 to n+7. In one aspect of this embodiment, 3-5 species are present for each of the PC, PE, PS, PG, and PI species and each of the 15-25 species are different. In another aspect of this embodiment, 3-5 species are present for each of the PC, PE, PS, PG, and PI species, each of the 15-25 species are different, and the R₃ and R₂ groups are repeated for each of the PC, PE, PS, PG, and PI species. For example, if 5 PC species have saturated acyl chains of 15 carbons in length for R₃ and unsaturated acyl chains of 12, 14, 16, 18, and 20 carbons in length for R₂, then the 5 PE, PS, PG, and PI species will also have saturated acyl chains of 15 carbons for R₃ and unsaturated (with same number of double and/or triple bonds at the same position(s)) acyl chains of 12, 14, 16, 18, and 20 carbons for R₂.

In one embodiment, phospholipid species included in the ULQS are PC, PE, PS, PG, and PI, at least one of R₄ (preferably all of R₄) is deuterium (D), R₃ is a saturated acyl chain of n carbons in length, R₂ is an unsaturated acyl chain of n-5 to n+7 carbons in length, and n is 15, 17, or 19, R₂ contains 1 or 2 double bonds (preferably 1 double bond) when the length of R₂ is from n-5 to n+1, R₂ contains 2 or 3 double bonds (preferably 3 double bonds) when the length of R₂ is from n+2 to n+4, and R₂ contains 3 or 4 double bonds (preferably 4 double bonds) when the length of R₂ is from n+5 to n+7. In one aspect of this embodiment, 3-5 species are present for each of the PC, PE, PS, PG, and PI species and each of the 15-25 species are different. In another aspect of this embodiment, 3-5 species are present for each of the PC, PE, PS, PG, and PI species, each of the 15-25 species are different, and the R₃ and R₂ groups are repeated for each of the PC, PE, PS, PG, and PI species. For example, if the 5 PC species have saturated acyl chains of 17 carbons in length for R₃ and unsaturated acyl chains of 14, 16, 18, 20, and 22 carbons in length for R₂, then the 5 PE, PS, PG, and PI species will also have saturated acyl chains of 17 carbons for R₃ and unsaturated (with the same number of double and/or triple bonds at the same position(s)) acyl chains of 14, 16, 18, 20, and 22 carbons for R₂.

In one embodiment, the phospholipid species included in the ULQS may be represented by the formula IB to IF below: 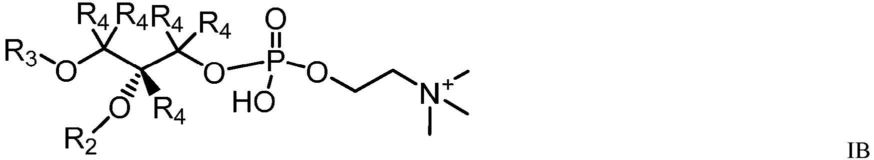 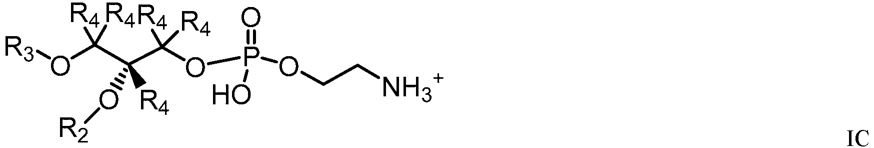 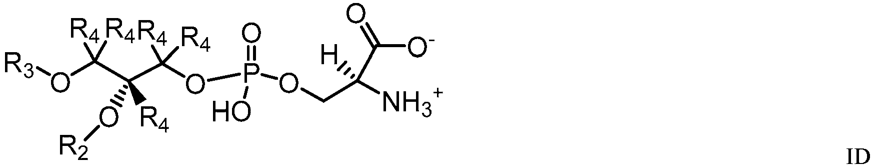 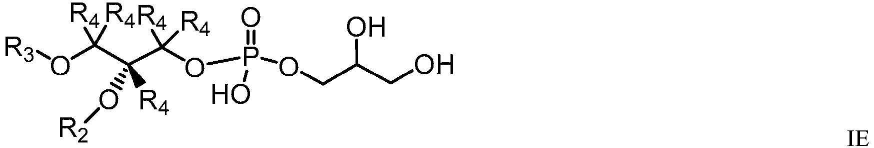 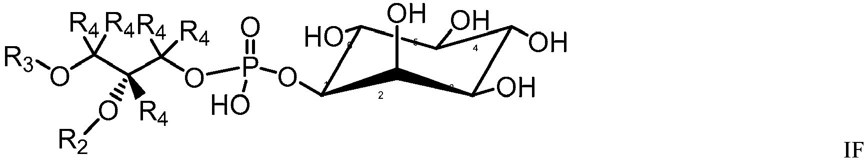 wherein for each of the compounds of formula IB to IF:
R₃ is a saturated acyl chain of the length n (where n is a 15, 17, or 19), and for each length of n at least one of R₄ (preferably all of R₄) is deuterium, and R₂ is represented by 5 different unsaturated acyl chains of: i) n-5, n-3, n+1, n+1, and n+3 carbons in length; ii) n-3, n-1, n+1, n+3, and n+5 carbons in length; or iii) n-1, n+1, n+3, n+5, and n+7 carbons in length; wherein the acyl chains of n-1, n+1, and n+3 contain 1 double bond, the acyl chain of n+3 contains 3 double bonds, and the acyl chain of n+5 and n+7 contains 4 double bonds.

In a specific embodiment, phospholipid species of the formula IB to IF incldued in the ULQS include those listed in Table 1 below, wherein at least one of R₄ (preferably all of R₄) is deuterium and PC is a compound of formula IB, PE is a compound of formula IC, PS is a compound of formula ID, PG is a compound of formula IE, and PI is a compound of formula IF, the underlined portion corresponds to the R₂ variable, and the double underlined portion corresponds to the R₃ variable.

| Table 1 | | | | |
|---|---|---|---|---|
| PC (17:0/14:1) | PE (17:0/14:1) | PS (17:0/14:1)) | PG (17:0/14:1)) | PI (17:0/14:1) |
| PC (17:0/16:1) | PE (17:0/16:1) | PS (17:0/16:1) | PG (17:0/16:1) | PI (17:0/16:1) |
| PC (17:0/18:1) | PE (17:0/18:1) | PS (17:0/18:1) | PG (17:0/18:1) | PI (17:0/18:1) |
| PC (17:0/20:3) | PE (17:0/20:3) | PS (17:0/20:3) | PG (17:0/20:3) | PI (17:0/20:3) |
| PC (17:0/22:4) | PE (17:0/22:4) | PS (17:0/22:4) | PG (17:0/22:4) | PI (17:0/22:4) |

### Lysophospholipids

In one embodiment, the lysophospholipid class includes a plurality of lysophospholipid species, wherein each lysophospholipid species contains an isotopic label. Any combination of lysophospholipid species described herein may be used in the ULQS. In one embodiment, the lysophospholipid class includes one or more of the following lysophospholipid species: lysophosphatidylcholine (LPC), lysophosphatidylethanolamine (LPE), lysophosphatidylsphoserine (LPS), lysophosphatidylglycerol (LPG), and lysophosphatidylinositol (LPI). In each of the foregoing, the acyl chain of the lysophospholipid is independently selected from a saturated C3 to C25 acyl chain or an unsaturated C3 to C25 acyl chain, containing from 1 to 6 double bonds.

In each of the foregoing, the acyl chains of the lysophospholipid species are independently selected from saturated or unsaturated C3 to C25 acyl chains, containing from 1 to 4 double bonds, such as from 1 to 3 or 1 to 2 double bonds.

In one embodiment, the acyl chains of the lysophospholipid species are each independently a saturated or unsaturated C3 to C6 acyl chain, a saturated or unsaturated C7 to C11 acyl chain, a saturated or unsaturated C12 to C18 acyl chain, a saturated or unsaturated C19 to C25 acyl chain. Such acyl chain, regardless of the length, includes both even and odd chain lengths and may be saturated or unsaturated. In a particular embodiment, the acyl chain of the lysophospholipid species are each selected such that the sum composition score for the acyl chain is an odd number. In a particular embodiment, the acyl chain of the lysophospholipid species are each independently an acyl chain of 3, 4, 5, 6, 7, 8, 9, 10, 11, 12, 13, 14, 15, 16, 17, 18, 19, 20, 21, 22, 23, 24, or 25 carbons, which are saturated or unsaturated. In another particular embodiment, the acyl chain of the lysophospholipid species are each independently an acyl chain of 10, 11, 12, 13, 14, 15, 16, 17, 18, 19, 20, 21, 22, 23, 24 or 25 carbons, which are saturated or unsaturated. In still another particular embodiment, the acyl chains of the lysophospholipid species are each independently an acyl chain of 13, 14, 15, 16, 17, 18, 19, 20, or 21 carbons, which are saturated or unsaturated.

In one embodiment, the acyl chain of the lysophospholipid species is saturated. In another embodiment, the acyl chains of the phospholipid species is unsaturated. When the acyl chain of the lysophospholipid species is unsaturated, the acyl chain may contain from 1 to 6, from 1 to 4 or from 1 to 3 double bonds. In one embodiment, the acyl chains is unsaturated, containing from 1 to 4, from 1 to 3 or from 1 to 2 double bonds. In certain embodiments, the acyl chain contains 2 or more double bonds when the acyl chain length is over 18 or over 20 (containing from example from 2 to 4 double bonds). The double bonds in such unsaturated acyl chains may be present in the cis or trans configuration, or a mixture of cis and trans configuration when more than 1 double bond is present.

In one embodiment, when multiple species of a particular lysophospholipid (for example, multiple lysophosphatidylcholine species) are present, the acyl chains on the various species are selected to include only saturated acyl chains. In one embodiment, when multiple species of a lysophospholipid are present, the acyl chain on the various species are selected to include both saturated and unsaturated acyl chains (for example, with 1 to 4 double bonds).

In certain embodiments, when multiple species of a particular lysophospholipid (for example, multiple lysophosphatidylcholine species) are present, the length of the acyl chains of the different species are different from one another.

In any of the foregoing embodiments, each lysophospholipid species contains at least one isotopically labeled H atom, preferably from 2 to 5 isotopically labeled H atoms. In preferred embodiments, the isotopically labeled H atom is deuterium (D).

In one embodiment of the foregoing, the phospholipids for inclusion in the ULQS are represented by the general formula II: 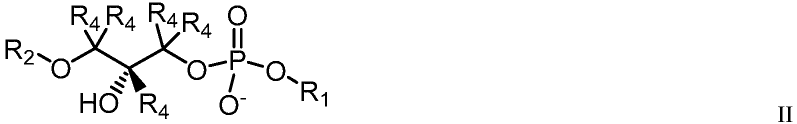 wherein:
R₁ is a head group moiety;
R₂ is a C2 to C24 saturated or unsaturated acyl chain; and
R₄ is independently H or an isotope of H, provided that at least one of R₄ is an isotope of H.

R₁ may be selected from any lysophospholipid head group known in the art. Suitable selections for R₁ include, but are not limited to, -(CH₂)ₙ-N(CH₃)₃, -(CH₂)ₙ-NH₃, -(CH₂)ₙ-C(H)(NH3)-C(O)-O-, -(CH₂)ₙ-CH(OH)-CH(OH), inositol, and H, where n is independently selected from 1 to 6, preferably 1 to 2.

Particularly preferred substituents for R₁ are of the formula (A), (B), (C), (D), and (E) as defined above for the phospholipids. When R₁ is (A), the lysophospholipid is lysophosphatidylcholine (LPC). When R₁ is (B), the lysophospholipid is lysophosphatidylethanolamine (LPE). When R₁ is (C), the lysophospholipid is lysophosphatidylserine (LPS). When R₁ is (D), the lysophospholipid is lysophosphatidylglycerol (LPG). When R₁ is (E), the lysophospholipid is lysophosphatidylinositol (LPI).

The descriptions below for R₂ and R₄ are applicable to phospholipid species containing any head group described herein.

R₂ is a saturated or unsaturated acyl chain from 3 to 25 carbons in length. In one embodiment, R₂ is an acyl chain from 3 to 6 carbons in length, 7 to 11 carbons in length, 12 to 18 carbons in length, or 19 to 25 carbons in length. In a particular aspect, R₂ is an acyl chain from 10 to 20 carbons in length. Such acyl chain, regardless of the length, includes both even and odd chain lengths and may be saturated or unsaturated. In a particular embodiment, R₂ is an acyl chain having an even number of carbon atoms. In one embodiment, R₂ is an acyl chain of 9, 10, 11, 12, 13, 14, 15, 16, 17, 18, 19, 20, 21, 22, 23, or 24 carbons, which are saturated or unsaturated. In one embodiment, R₂ is an acyl chain of 2, 3, 4, 5, 6, 7, or 8 carbons, which are saturated or unsaturated. In another embodiment, R₂ is an acyl chain of 12, 13, 14, 15, 16, 17, 18, 19, 20, or 21 carbons, which is saturated or unsaturated. In another embodiment, R₂ is an acyl chain of 14, 15, 16, 17, 18, 19, or 20 carbons, which are saturated or unsaturated. In a preferred embodiment, R₂ is a saturated acyl chain when the acyl chains of R₂ are of the lengths specified above.

In one embodiment, R₂ is a saturated acyl chain. In another embodiment, R₂ is an unsaturated acyl chain. In a preferred embodiment, R₂ is a saturated acyl chain. When R₂ is an unsaturated acyl chain, the acyl chain may contain from 1 to 6, from 1 to 4 or from 1 to 3 double bonds. In one embodiment, R₂ is an unsaturated acyl chain; containing from 1 to 4, from 1 to 3 or from 1 to 2 double bonds. The double bonds in such unsaturated acyl chains may be present in the cis or trans configuration, or a mixture of cis and trans configuration when more than 1 double bond is present.

In one embodiment, when multiple species of a lysophospholipid are present, the R₂ groups on the various species are selected to include only saturated acyl chains and acyl chains with odd numbers of carbon atoms.

In one embodiment of any of the foregoing, the isotopically labeled H is deuterium (D). In one embodiment of any of the foregoing, at least 1, at least 2, at least 3, at least 4 or all 5 of R₄ are isotopically labeled H, preferable deuterium (D).

In certain embodiments, at least a subset of the lysophospholipid species included in the ULQS have different R₂ groups. In certain embodiments, the lysophospholipid species included in the ULQS have each of the head groups of formula (A), (B), (C), (D), and (E). In certain embodiments, the lysophospholipid species included in the ULQS have each of the head groups of formula (A), (B), (C), (D), and (E), and the R₂ group of the lysophospholipid species is saturated. In certain embodiments, the lysophospholipid species included in the ULQS have each of the head groups of formula (A), (B), (C), (D), and (E), the R₂ group of the lysophospholipid species is saturated and the R₂ group contains acyl chains with odd carbon numbers.

In certain embodiments, the lysophospholipid species included in the ULQS have each of the head groups of formula (A), (B), (C), (D), and (E) and the R₂ groups are repeated for lysophospholipid species with different head groups and different for lysophospholipid species with the same head group. For example, a set of 20 lysophospholipid species suitable for inclusion in the ULQS may be represented by the formula II, wherein there are 4 lysophospholipid species for each head group A to E above, at least one of R₄ (preferably all of R₄) is deuterium, and R₂ for each of the 4 lysophospholipid species of each head group A to E is a saturated acyl chain of 13, 15, 17, and 19 carbons in length.

In a one embodiment, lysophospholipid species included in the ULQS include LPC, LPE, LPS, LPG, and LPI, R₂ is a saturated acyl chain from 10 to 25 carbons in length, and at least one of R₄ (preferably all of R₄) are deuterium (D). In one aspect of this embodiment, 3-5 species are present for each of LPC, LPE, LPS, LPG, and LPI and each of the 15-25 species are different. In another aspect of this embodiment, 3-5 species are present for each of LPC, LPE, LPS, LPG, and LPI, each of the 15-25 individual species are different, and the R₂ groups are repeated for each of LPC, LPE, LPS, LPG, and LPI. For example, if three LPC species have saturated acyl chains of 12, 14 and 16 carbons in length, then the 3 LPE, LPS, LPG, and LPI species will also have saturated acyl chains of 12, 14 and 16 carbons.

In a one embodiment, lysophospholipid species included in the ULQS include LPC, LPE, LPS, LPG, and LPI, R₂ is a saturated acyl chain from 11, 13, 15, 17, 19, 21, or 23 carbons in length, at least one of R₄ (preferably all of R₄) are deuterium (D). In one aspect of this embodiment, 3-5 species are present for each of LPC, LPE, LPS, LPG, and LPI and each of the 15-25 species are different. In another aspect of this embodiment, 3-5 species are present for each of LPC, LPE, LPS, LPG, and LPI, each of the 15-25 individual species are different, and the R₂ groups are repeated for each of LPC, LPE, LPS, LPG, and LPI. For example, if three LPC species have saturated acyl chains of 15, 17 and 19 carbons, then the 3 LPE, LPS, LPG, and LPI species will also have saturated acyl chains of 15, 17 and 19 carbons.

In a one embodiment, lysophospholipid species included in the ULQS include LPC, LPE, LPS, LPG, and LPI, R₂ is a saturated acyl chain from 13, 15, 17, 19, or 21, carbons in length, at least one of R₄ (preferably all of R₄) are deuterium (D). In one aspect of this embodiment, 3-5 species are present for each of LPC, LPE, LPS, LPG, and LPI and each of the 15-25 species are different. In another aspect of this embodiment, 3-5 species are present for each of LPC, LPE, LPS, LPG, and LPI, each of the 15-25 individual species are different, and the R₂ groups are repeated for each of LPC, LPE, LPS, LPG, and LPI. For example, if three LPC species have saturated acyl chains of 13, 17 and 21 carbons, then the 3 LPE, LPS, LPG, and LPI species will also have saturated acyl chains of 13, 17 and 21 carbons.

In one embodiment, the lysophospholipid species included in the ULQS may be represented by the formula IIB to IIF below: 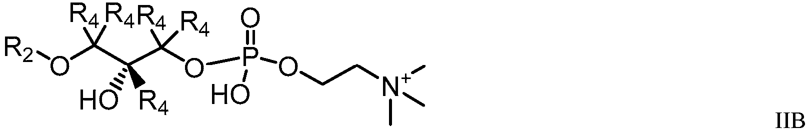 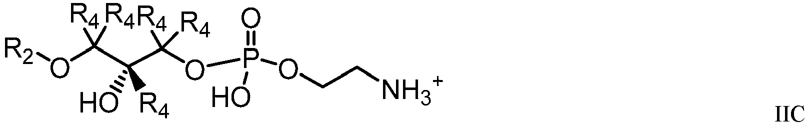 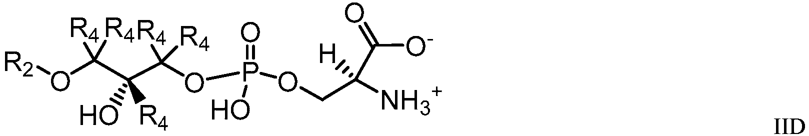 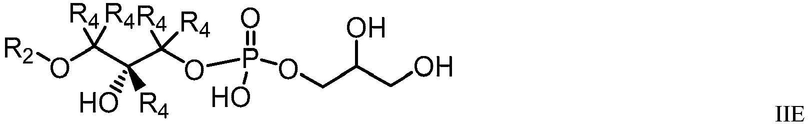 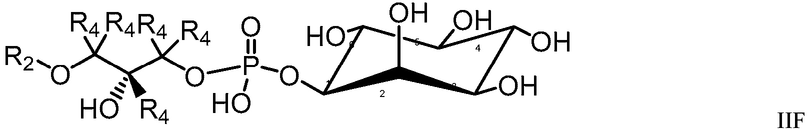 wherein: at least one of R₄ (preferably all of R₄) is deuterium, and R₂ is represented by 3 different saturated acyl chains of: i) 13, 15, and 17 carbons in length; ii) 15, 17 and 19 carbons in length; or iii) 17, 19, or 21 carbons in length.

In a specific embodiment, lysophospholipid species of the formula IIA-F included in the ULQS include those listed in Table 2 below, wherein at least one of R₄ (preferably all of R₄) is deuterium and LPC is a compound of formula A, LPE is a compound of formula B, LPS is a compound of formula C, LPG is a compound of formula D, and LPI is a compound of formula E and the underlined portion corresponds to the R₂ variable.

| Table 2 | | | | |
|---|---|---|---|---|
| LPC (15:0) | LPE (15:0) | LPS (15:0) | LPG (15:0) | LPI (15:0) |
| LPC (17:0) | LPE (17.0) | LPS (17:0) | LPG (17.0) | LPI (17:0) |
| LPC (19:0) | LPE (19:0) | LPS (19:0) | LPG (19:0) | LPI (19:0) |

### Cholesterol esters

In one embodiment, the cholesterol ester class includes a plurality of cholesterol ester species, wherein each cholesterol ester species contains an isotopic label. Any combination of cholesterol ester species described herein may be used in the ULQS. In one embodiment, the cholesterol ester class includes a plurality of cholesterol ester species, wherein each cholesterol ester species contains at least one isotopically labeled H atom, preferably from 2 to 9 isotopically labeled H atoms. In preferred embodiments, the isotopically labeled H atom is deuterium (D).

In one embodiment, the cholesterol esters for inclusion in the ULQS are represented by the general formula III, or a pharmaceutically acceptable salt thereof: 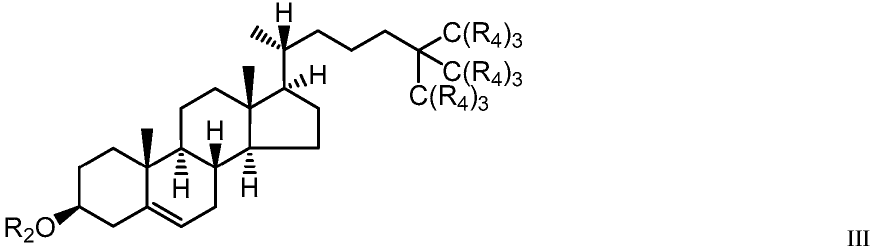 wherein:
R₂ is a C9 to C29 saturated or unsaturated acyl chain; and
R₄ is independently H or an isotope of H, provided that at least one of R₄ is an isotope of H.

R₂ is a saturated or unsaturated acyl chain from 2 to 29 carbons in length. In one embodiment, R₂ is an acyl chain from 2 to 5 carbons in length, an acyl chain from 6 to 10 carbons in length, an acyl chain from 10 to 20 carbons in length, or an acyl chain from 21 to 29 carbons in length. In a particular aspect, R₂ is an acyl chain from 10 to 20 or 21 to 29 carbons in length. Such acyl chain, regardless of the length, includes both even and odd chain lengths and may be saturated or unsaturated. In a particular embodiment, the acyl chains of the cholesterol ester species are each selected such that the sum composition score of the acyl chain is an even number. In another embodiment, R₂ is an acyl chain of 3, 4, 5, 6, 7, 8, 9, 10, 11, 12, 13, 14, 15, 16, 17, 18, 19, 20, 21, 22, 23, 24, 25, 26, 27, 28, or 29 carbons, which are saturated or unsaturated. In another embodiment, R₂ is an acyl chain of 10, 11, 12, 13, 14, 15, 16, 17, 18, 19, 20, 21, 22, 23, or 24 carbons, which is saturated or unsaturated. In another embodiment, R₂ is an acyl chain of 14, 15, 16, 17, 18, 19, 20, 21, or 22 carbons, which are saturated or unsaturated.

In one embodiment, R₂ is a saturated acyl chain. In another embodiment, R₂ is an unsaturated acyl chain. When R₂ is an unsaturated acyl chain, the acyl chain may contain from 1 to 6, from 1 to 4 or from 1 to 3 double bonds. In one embodiment, R₂ is an unsaturated acyl chain; containing from 1 to 4, from 1 to 3 or from 1 to 2 double bonds. In certain embodiments, R₂ is contains 2 or more double bonds when the acyl chain length is over 18 or over 20 (containing from example from 2 to 4 double bonds). The double bonds in such unsaturated acyl chains may be present in the cis or trans configuration, or a mixture of cis and trans configuration when more than 1 double bond is present.

In one embodiment, when multiple species of a cholesterol ester are present, the R₂ groups on the various species are selected to include both saturated and unsaturated acyl chains (for example, with 1 to 4 double bonds). In one embodiment, when multiple species of a cholesterol ester are present, the R₂ groups on the various species are selected to include only unsaturated acyl chains (for example, with 1 to 6 or 1 to 4 double bonds). In one embodiment, when multiple species of a cholesterol ester are present, the R₂ groups on the various species are selected to include only unsaturated acyl chains (for example, with 1 to 4 double bonds) and includes acyl chains with 2 to 4 double bonds.

In one embodiment of any of the foregoing, the isotopically labeled H is deuterium (D). In one embodiment of any of the foregoing, at least 1, at least 2, at least 3, at least 4, at least 5. at least 6, at least 7, at least 8, or all 9 of R₄ are isotopically labeled H, preferably deuterium (D).

In certain embodiments, at least a subset of the cholesterol ester species included in the ULQS have different R₂ groups. In certain embodiments, all of the cholesterol ester species included in the ULQS have different R₂ groups and the R₂ groups are unsaturated acyl chains (for example, with 1 to 6 or 1 to 4 double bonds). In certain embodiments, all of the cholesterol ester species included in the ULQS have different R₂ groups and the R₂ groups are unsaturated acyl chains (for example, with 1 to 6 or 1 to 4 double bonds) and includes acyl chains with 2 to 4 double bonds (for example, when the acyl chain length is 20 carbons or greater).

For example, if the ULQS contains 5 cholesterol ester species, the species may be represented by the formula III, where R₂ is a C10 to C20 unsaturated acyl chain and each of R₄ is deuterium.

In a specific embodiment, suitable cholesterol ester species suitable for inclusion in the ULQS include those listed in Table 3 below (with reference to formula III, wherein at least one of R₄, preferably all of R₄, is deuterium, and the underlined portion corresponds to the R₂ variable).

| Table 3 | | | | |
|---|---|---|---|---|
| CE (14:1) | CE (16:1) | CE (18:1) | CE (20:3) | CE (22:4) |

### Triacylglycerols

In one embodiment, the triacylglycerol class includes a plurality of triacylglycerol species, wherein each triacylglycerol species contains an isotopic label. Any combination of triacylglycerol species described herein may be used in the ULQS. In one embodiment, the acyl chains of the triacylglycerol species are independently selected from saturated C3 to C30 acyl chains or unsaturated C3 to C30 acyl chains, containing from 1 to 6 double bonds, such as from 1 to 4 or 1 to 2 double bonds.

In one embodiment, the acyl chains of the triacylglycerol species are each independently a saturated or unsaturated C3 to C5 acyl chain, a saturated or unsaturated C6 to C10 acyl chain, a saturated or unsaturated C11 to C20 acyl chain, and a saturated or unsaturated C21 to C30 acyl chain. Such acyl chains, regardless of the length, includes both even and odd chain lengths and may be saturated or unsaturated. In a particular embodiment, the acyl chains of the triacylglycerol species are each selected such that the sum composition score for the acyl chains is an odd number. In a particular embodiment, the acyl chains of the triacylglycerol species are each independently an acyl chain of 3, 4, 5, 6, 7, 8, 9, 10, 11, 12, 13, 14, 15, 16, 17, 18, 19, 20, 21, 22, 23, 24, 25, 26, 27, 28, 29, or 30 carbons, which are saturated or unsaturated. In another particular embodiment, the acyl chains of the triacylglycerol species are each independently an acyl chain of 12, 13, 14, 15, 16, 17, 18, 19, 20, 21, 22, or 23, which are saturated or unsaturated. In still another particular embodiment, the acyl chains of the triacylglycerol species are each independently an acyl chain of 13, 14, 15, 16, 17, 18, 19, 20, 21, or 22, carbons, which are saturated or unsaturated.

In one embodiment, one or more of the acyl chains of the triacylglycerol species are saturated. In another embodiment, one or more of the acyl chains of the triacylglycerol species are unsaturated. In still another embodiment, one of the acyl chains is saturated and two of the acyl chains are unsaturated. In still another embodiment, two of the acyl chains are saturated and one of the acyl chains is unsaturated. In still another embodiment, all three acyl chains are unsaturated. When one or more of the acyl chains of the triacylglycerol species are unsaturated, the acyl chain may contain from 1 to 6, from 1 to 4, from 1 to 3, or from 1 to 2 double bonds. In one embodiment, at least one, two, or all three of the acyl chains are unsaturated, containing from 1 to 4, from 1 to 3 or from 1 to 2 double bonds. In certain embodiments, at least one of the acyl chains contains 2 or more double bonds when the acyl chain length is over 18 (containing from example from 2 double bonds). The double bonds in such unsaturated acyl chains may be present in the cis or trans configuration, or a mixture of cis and trans configuration when more than 1 double bond is present.

In one embodiment, when multiple species of a particular triacylglycerol are present, the acyl chains on the various species are selected to include both saturated and unsaturated acyl chains (for example, with 1 to 4 double bonds) or the acyl chains on the various species are selected to include only unsaturated acyl chains (for example, with 1 to 4 double bonds). In one embodiment, when multiple species of a triacylglycerol are present, the acyl chains on the various species are selected to include both saturated and unsaturated acyl chains (for example, with 1 and 2 double bonds) and includes acyl chains with 2 double bonds when the acyl chain length is over 18. In one embodiment, when multiple species of a triacylglycerol are present, the acyl chains on the various species are selected to include only unsaturated acyl chains (for example, with 1 and 2 double bonds) and includes acyl chains with 2 double bonds when the acyl chain length is over 18.

In certain embodiments, when multiple triacylglycerol species are present, the length of one acyl chain is different from the length of the other two acyl chains (which are preferably the same). In certain embodiments, when multiple triacylglycerol species are present, the length of one acyl chain is different from the length of the other two acyl chains (which are preferably the same) and at least one acyl chains is unsaturated (containing for example 1 to 4 double bonds) and at least one acyl chain is saturated (contains no double bonds). In certain embodiments, when multiple triacylglycerol species are present, the length of one acyl chain is different from the length of the other two acyl chains (which are preferably the same) and each of the acyl chains of the same length are saturated and the acyl chain of a different length is unsaturated (containing for example 1 to 2 double bonds and 2 double bonds when the acyl chain length is greater than 18 carbons). In certain embodiments, when multiple triacylglycerol species are present, the length of one acyl chain is different from the length of the other two acyl chains (which are preferably the same) and each of the acyl chains are unsaturated.

In certain embodiments, when multiple triacylglycerol species are present, the sum composition score of the different species are different from one another. In certain embodiments, when multiple triacylglycerol species are present, the sum composition scores of the different species are different from one another and one or two of the acyl chains is unsaturated (containing for example 1 to 4 double bonds, particularly 2 to 3 double bonds when the acyl chain length is over 18) and on or two acyl chains are saturated. In one embodiment, the sum composition score for the acyl chains of the different species is an odd number.

In any of the foregoing embodiments, the triacylglycerol contains at least one isotopically labeled H atom, preferably from 2 to 5 isotopically labeled H atoms. In preferred embodiments, the isotopically labeled H atom is deuterium (D).

In one embodiment of the foregoing, the triacylglycerols for inclusion in the ULQS are represented by the general formula IV, or a pharmaceutically acceptable salt thereof: 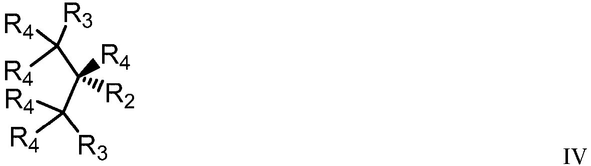 wherein:
R₂ is a C3 to C30 saturated or unsaturated acyl chain;
R₃ are each independently a C3 to C25 saturated or unsaturated acyl chain; and
R₄ is independently H or an isotope of H, provided that at least one of R₄ is an isotope of H.

R₂ is a saturated or unsaturated acyl chain from 3 to 30 carbons in length. In one embodiment, R₂ is an acyl chain from 3 to 5 carbons in length, an acyl chain from 6 to 10 carbons in length, an acyl chain from 10 to 20 carbons in length, or an acyl chain from 21 to 30 carbons in length. In a particular aspect, R₂ is an acyl chain from 10 to 25 carbons in length. In another particular aspect, R₂ is an acyl chain from 12 to 23 carbons in length. Such acyl chains, regardless of the length, include both even and odd chain lengths and may be saturated or unsaturated. In a particular embodiment, R₂ is an acyl chain having an odd number of carbon atoms. In another embodiment, R₂ is an acyl chain of 3, 4, 5, 6, 7, 8, 9, 10, 11, 12, 13, 14, 15, 16, 17, 18, 19, 20, 21, 22, 23, 24, 25, 26, 27, 28, 29, or 30 carbons, which are saturated or unsaturated. In another embodiment, R₂ is an acyl chain of 12, 13, 14, 15, 16, 17, 18, 19, 20, 21, 22, or 23 carbons, which is saturated or unsaturated. In another embodiment, R₂ is an acyl chain of 13, 14, 15, 16, 17, 18, 19, 20, 21, or 22 carbons, which are saturated or unsaturated. In a preferred embodiment, acyl chains of the foregoing lengths are unsaturated containing from 1 to 6, such as from 1 to 4 or from 1 to 2, double bonds.

In one embodiment, R₂ is a saturated acyl chain. In another embodiment, R₂ is an unsaturated acyl chain. When R₂ is an unsaturated acyl chain, the acyl chain may contain from 1 to 6, from 1 to 4 or from 1 to 3 double bonds. In one embodiment, R₂ is an unsaturated acyl chain, containing from 1 to 4, from 1 to 3 or from 1 to 2 double bonds. In certain embodiments, R₂ is contains 2 to 4 double bonds when the acyl chain length is over 18 (containing from example from 2 double bonds). The double bonds in such unsaturated acyl chains may be present in the cis or trans configuration, or a mixture of cis and trans configuration when more than 1 double bond is present.

In one embodiment, when multiple triacylglycerol species are present, the R₂ groups on the various species are selected to include both saturated and unsaturated acyl chains (for example, with 1 to 4 double bonds). In one embodiment, when multiple triacylglycerol species are present, the R₂ groups on the various species are selected to include only unsaturated acyl chains (for example, with 1 to 4 double bonds). In one embodiment, when multiple triacylglycerol species are present, the R₂ groups on the various species are selected to include only unsaturated acyl chains (for example, with 1 to 4 double bonds) and includes acyl chains with 2 to 4 double bonds, preferably 2 double bonds, when the acyl chain length is greater than 18 carbons.

R₃ is a saturated or unsaturated acyl chain from 3 to 25 carbons in length. In one embodiment, R₃ is an acyl chain from 3 to 5 carbons in length, an acyl chain from 6 to 10 carbons in length, an acyl chain from 10 to 20 carbons in length, or an acyl chain from 21 to 25 carbons in length. In a particular aspect, R₃ is an acyl chain from 10 to 20 carbons in length. In another particular aspect, R₃ is an acyl chain from 12 to 20 carbons in length. Such acyl chains, regardless of the length, include both even and odd chain lengths and may be saturated or unsaturated. In a particular embodiment, R₃ is an acyl chain having an even number of carbon atoms. In another embodiment, R₃ is an acyl chain of 3, 4, 5, 6, 7, 8, 9, 10, 11, 12, 13, 14, 15, 16, 17, 18, 19, 20, 21, 22, 23, 24, 25, 26, 27, 28, 29, or 30 carbons, which are saturated or unsaturated. In another embodiment, R₃ is an acyl chain of 10, 11, 12, 13, 14, 15, 16, 17, 18, 19, 20, 21, or 22, carbons, which is saturated or unsaturated. In another embodiment, R₃ is an acyl chain of 14, 15, 16, 17, or 18 carbons, which are saturated or unsaturated. In a preferred embodiment, acyl chains of the foregoing lengths are saturated when the acyl chain is 14 carbons or less and unsaturated when the acyl chain is greater than 15 carbons (for example, containing from 1 to 4 double bonds.

In one embodiment, R₃ is a saturated acyl chain. In another embodiment, R₃ is an unsaturated acyl chain. When R₃ is an unsaturated acyl chain, the acyl chain may contain from 1 to 4, such as 1 to 2 double bonds. In one embodiment, R₃ is an unsaturated acyl chain, containing from 1 to 2 double bonds (for example, when the acyl chain is greater than 17 carbons in length). In one embodiment, R₃ is an unsaturated acyl chain, containing 1 double bond when the acyl chain is greater than 17 carbons in length. The double bonds in such unsaturated acyl chains may be present in the cis or trans configuration, or a mixture of cis and trans configuration when more than 1 double bond is present.

In one embodiment, when multiple triacylglycerol species are present, the R₃ groups on the various species are selected to include both saturated and unsaturated acyl chains (for example, with 1 to 2 double bonds). In one embodiment, when multiple triacylglycerol species are present, the R₃ groups on the various species are selected to include only saturated acyl chains.

In one embodiment of any of the foregoing, the isotopically labeled H is deuterium (D). In one embodiment of any of the foregoing, at least 1, at least 2, at least 3, at least 4 or all 5 of R₄ are isotopically labeled H, preferable deuterium (D).

In certain embodiments, at least a subset of the triacylglycerol species included in the ULQS have the same R₃ groups and different R₂ groups. In certain embodiments, all of the triacylglycerol species included in the ULQS have the same R₃ groups and different R₂ groups, the R₃ groups are saturated or unsaturated acyl chains (for example, with 1 to 2 double bonds), and the R₂ groups are saturated or unsaturated acyl chains (for example, with 1 to 4 double bonds). In certain embodiments, all of the triacylglycerol species included in the ULQS have the same R₃ groups and different R₂ groups, the R₃ groups are saturated or unsaturated acyl chains (for example, with 1 to 2 double bonds), and the R₂ groups are saturated or unsaturated acyl chains (for example, with 1 to 4 double bonds) and includes acyl chains with 2 to 3 double bonds when the acyl chain length is greater than 18. In certain embodiments, all of the triacylglycerol species included in the ULQS have the same R₃ groups and different R₂ groups, the R₃ groups are saturated when the acyl chain length is less than 18 and unsaturated when the acyl chain length is 18 or greater (for example, with 1 to 2 double bonds), and the R₂ groups are saturated when the acyl chain length is less than 15 or unsaturated when the acyl chain length is 15 or greater (for example, with 1 to 4 double bonds) and includes acyl chains with 2 double bonds when the acyl chain length is greater than 18.

In one embodiment, the triacylglycerol species included in the ULQS include those species wherein each R₃ is an acyl chain of n atoms in length, n is 10 to 22, R₂ is an acyl chain from n-3 to n+5 carbon atoms, wherein R₃ is saturated or unsaturated, R₂ is saturated or unsaturated (containing 1 or 2 double bonds) when the length of R₂ is from n-3 to n+1 carbon atoms and R₂ contains 2 or 3 double bonds (preferably 2 double bonds) when the length of R₂ is from n+2 to n+5 carbon atoms.

In one embodiment, the triacylglycerol species included in the ULQS include those species wherein R₃ is independently an acyl chain of n atoms in length, n is 12, 14, 16, 18, 20, or 22, R₂ is an acyl chain from n-3 to n+5 carbon atoms, wherein R₃ is saturated when n is 12, 14, or 16 and unsaturated when n is 18, 20, or 22 (containing from 1 to 2 double bonds, preferably 1 double bond), R₂ contains 1 or 2 double bonds when the length of R₂ is from n-3 to n+1 carbon atoms and R₂ contains 2 or 3 double bonds (preferably 2 double bonds) when the length of R₂ is from n+2 to n+5 carbon atoms.

In one embodiment, the triacylglycerol species suitable for inclusion in the ULQS include those species wherein R₃ is independently an acyl chain of n carbon atoms in length, n is 14, 16, or 18, R₂ is an acyl chain from n-3 to n+5 carbon atoms, wherein R₃ is saturated when n is 14 or 16 and unsaturated when n is 18 (containing from 1 to 2 double bonds, preferably 1 double bond), R₂ contains 1 or 2 double bonds when the length of R₂ is from n-3 to n+1 carbon atoms and R₂ contains 2 or 3 double bonds (preferably 2 double bonds) when the length of R₂ is from n+2 to n+5 carbon atoms.

In one embodiment, the triacylglycerol species suitable for inclusion in the ULQS may be represented by the formula IV:
wherein:
at least one of R₄ (preferably all of R₄) is deuterium;
R₃ are each a saturated acyl chain of 12, 14 or 16 carbons in length, or an unsaturated acyl chain of 18 or 20 carbons in length containing 1 double bond; and
R₂ is a saturated or unsaturated acyl chain of 11, 13, 15, 17, 19, 21, 22, or 23 carbons in length, wherein R₂ is saturated when R₂ is 11 or 13 carbons in length, R₂ contains 1 double bond when R₂ is 15 or 17 carbons in length, and R₂ contains 2 double bonds when R₂ is 19, 21, or 23 carbons in length.

In a particular aspect of this embodiment, the triacylglycerol species suitable for inclusion in the ULQS consists of 9 species wherein: R₃ is 12:0 and R₂ is 11:0, 13:1, and 15:1; R₃ is 14:0 and R₂ 13:0, 15:1, and 17:1; and R₃ is 16:0 and R₂ is 15:1, 17:1, and 19:2.

In a particular aspect of this embodiment, the triacylglycerol species suitable for inclusion in the ULQS consists of 9 species wherein: R₃ is 14:0 and R₂ is 13:0, 15:1, and 17:1; R₃ is 16:0 and R₂ is 15:1, 17:1, and 19:2; and R₃ is 18:1 and R₂ is 17:1, 19:2, and 21:2.

In a particular aspect of this embodiment, the triacylglycerol species suitable for inclusion in the ULQS consists of 9 species wherein: R₃ is 16:0 and R₂ is 15:1, 17:1, and 19:2; R₃ is 18:1 and R₂ is 17:1, 19:2, and 21:2; and R₃ is 20:1 and R₂ is 19:2, 21:2, and 23:2.

In a specific embodiment, suitable triacylglycerol species suitable for inclusion in the ULQS include those listed in Table 4 below (with reference to formula IV, wherein at least one of R₄ (preferably all of R₄) is deuterium, the underlined portion corresponds to the R₂ variable and the double underlined portion correspond to the R₃ variable).

| Table 4 | | |
|---|---|---|
| TAG (14:0/13:0/14:0) | TAG (16:0/15:1/16:0) | TAG (18:1/17:1/18:1) |
| TAG (14:0/15:1/14:0) | TAG (16:0/17:1/16:0) | TAG (18:1/19:2/18:1) |
| TAG (14:0/17:1/14:0) | TAG (16:0/19:2/16:0) | TAG (18:1/21:2/18:1) |

### Diacylglycerols

In one embodiment, the diacylglycerol class includes a plurality of diacylglycerol species, wherein each diacylglycerol species contains an isotopic label. Any combination of diacylglycerol molecules known in the art may be used. In one embodiment, the acyl chains of the diacylglycerol species are independently selected from saturated C3 to C30 acyl chains or unsaturated C3 to C30 acyl chains, containing from 1 to 6 double bonds, such as from 1 to 4 or 1 to 2 double bonds.

In one embodiment, both of the acyl chains of the diacylglycerol species are each independently a saturated or unsaturated C3 to C6 acyl chain, a saturated or unsaturated C7 to C11 acyl chain, a saturated or unsaturated C12 to C21 acyl chain, a saturated or unsaturated C22 to C30 acyl chain. Such acyl chains, regardless of the length, includes both even and odd chain lengths and may be saturated or unsaturated. In a particular embodiment, the acyl chains of the diacylglycerol species are each selected such that the sum composition score for the acyl chains is an odd number. In a particular embodiment, both of the acyl chains of the diacylglycerol species are each independently an acyl chain of 3, 4, 5, 6, 7, 8, 9, 10, 11, 12, 13, 14, 15, 16, 17, 18, 19, 20, 21, 22, 23, 24, 25, 26, 27, 28, 29, or 30 carbons, which are saturated or unsaturated. In another particular embodiment, both of the acyl chains of the diacylglycerol species are each independently an acyl chain of 10, 11, 12, 13, 14, 15, 16, 17, 18, 19, 20, 21, 22, 23, 24 or 25 carbons, which are saturated or unsaturated. In still another particular embodiment, both of the acyl chains of the diacylglycerol species are each independently an acyl chain of 15, 16, 17, 18, 19, 20, 21, 22, or 23 carbons, which are saturated or unsaturated.

In one embodiment, one or both of the acyl chains of the diacylglycerol species are saturated. In another embodiment, one or both of the acyl chains of the diacylglycerol species are unsaturated. In still another embodiment, one of the acyl chains is saturated and one of the acyl chains is unsaturated. When one or both of the acyl chains of the diacylglycerol species are unsaturated, the acyl chain may contain from 1 to 6, from 1 to 4, from 1 to 3, or from 1 to 2 double bonds. In one embodiment, at least one of the acyl chains is unsaturated, containing from 1 to 6, 1 to 4, from 1 to 3 or from 1 to 2 double bonds. In certain embodiments, at least one of the acyl chains contains 2 or more double bonds when the acyl chain length is over 18 (containing from example from 3 to 4 double bonds). The double bonds in such unsaturated acyl chains may be present in the cis or trans configuration, or a mixture of cis and trans configuration when more than 1 double bond is present.

In one embodiment, when multiple diacylglycerol species are present, the acyl chains on the various species are selected to include both saturated and unsaturated acyl chains (for example, with 1 to 4 double bonds). In one embodiment, when multiple diacylglycerol species are present, the acyl chains on the various species are selected to include both saturated and unsaturated acyl chains (for example, with 1 to 4 double bonds) and includes acyl chains with 3 to 4 double bonds (preferably when the when the acyl chain length is over 18).

In certain embodiments, when multiple diacylglycerol species are present, the length of one acyl chain is different from the length of the other acyl chain. In certain embodiments, when multiple diacylglycerol species are present, the length of one acyl chain is different from the length of the other acyl chain and one acyl chains is unsaturated (containing for example 1 to 4 double bonds) and one acyl chain is saturated (contains no double bonds). In certain embodiments, when multiple diacylglycerol species are present, the length of one acyl chain is different from the length of the other acyl chain and one acyl chains is unsaturated and contains 1 double bond when the acyl chain length is less than 18 and 3 to 4 double bonds when the acyl chain length is greater than 18.

In certain embodiments, when multiple diacylglycerol species are present, the sum composition score of the different species are different from one another. In certain embodiments, when multiple diacylglycerol species are present, the sum composition scores of the different species are different from one another and one of the acyl chains is unsaturated (containing for example 1 to 4 double bonds, particularly 3 to 4 double bonds when the acyl chain length is over 18) and the other acyl chain is saturated. In one embodiment, the sum composition score for the acyl chains of the different species is an odd number.

In any of the foregoing embodiments, the diacylglycerol contains at least one isotopically labeled H atom, preferably from 2 to 5 isotopically labeled H atoms. In preferred embodiments, the isotopically labeled H atom is deuterium (D).

In one embodiment of the foregoing, the diacylglycerols for inclusion in the ULQS are represented by the general formula V: 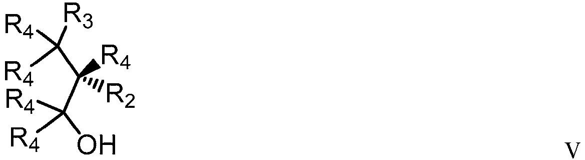 wherein:
R₂ is a C3 to C30 saturated or unsaturated acyl chain;
R₃ is a C3 to C25 saturated or unsaturated acyl chain; and
R₄ is independently H or an isotope of H, provided that at least one of R₄ is an isotope of H.

R₂ is a saturated or unsaturated acyl chain from 3 to 30 carbons in length. In one embodiment, R₂ is an acyl chain from 3 to 6 carbons in length, an acyl chain from 7 to 11 carbons in length, an acyl chain from 12 to 21 carbons in length, or an acyl chain from 22 to 30 carbons in length. In a particular aspect, R₂ is an acyl chain from 10 to 20 or 21 to 25 carbons in length. In another particular aspect, R₂ is an acyl chain from 12 to 24 carbons in length. Such acyl chains, regardless of the length, include both even and odd chain lengths and may be saturated or unsaturated. In a particular embodiment, R₂ is an acyl chain having an even number of carbon atoms. In another embodiment, R₂ is an acyl chain of 3, 4, 5, 6, 7, 8, 9, 10, 11, 12, 13, 14, 15, 16, 17, 18, 19, 20, 21, 22, 23, 24, 25, 26, 27, 28, 29, or 30 carbons, which are saturated or unsaturated. In another embodiment, R₂ is an acyl chain of 10, 11, 12, 13, 14, 15, 16, 17, 18, 19, 20, 21, 22, 23, or 24 carbons, which is saturated or unsaturated. In another embodiment, R₂ is an acyl chain of 14, 15, 16, 17, 18, 19, 20, 21, or 22 carbons, which are saturated or unsaturated. In a preferred embodiment, acyl chains of the foregoing lengths are unsaturated containing from 1 to 6, such as from 1 to 4 or from 1 to 2, double bonds. In a preferred embodiment, acyl chains of the foregoing lengths are unsaturated containing 1 double bond when the acyl chain length is less than 18, and 3 to 4 double bonds when the acyl chain length is over 18.

In one embodiment, R₂ is a saturated acyl chain. In another embodiment, R₂ is an unsaturated acyl chain. When R₂ is an unsaturated acyl chain, the acyl chain may contain from 1 to 6, from 1 to 4 or from 1 to 3 double bonds. In one embodiment, R₂ is an unsaturated acyl chain, containing from 1 to 4, from 1 to 3 or from 1 to 2 double bonds. In certain embodiments, R₂ is contains 2 or more double bonds when the acyl chain length is over 18 (containing from example from 2 to 4 double bonds). In certain embodiments, R₂ contains 3 double bonds when the acyl chain length is over 19. In certain embodiments, R₂ contains 4 double bonds when the acyl chain length is over 21. The double bonds in such unsaturated acyl chains may be present in the cis or trans configuration, or a mixture of cis and trans configuration when more than 1 double bond is present.

In one embodiment, when multiple diacylglycerol species are present, the R₂ groups on the various species are selected to include both saturated and unsaturated acyl chains (for example, with 1 to 4 double bonds). In one embodiment, when multiple diacylglycerol species are present, the R₂ groups on the various species are selected to include only unsaturated acyl chains (for example, with 1 to 4 double bonds). In one embodiment, when multiple diacylglycerol species are present, the R₂ groups on the various species are selected to include only unsaturated acyl chains (for example, with 1 to 4 double bonds) and includes acyl chains with 2 to 4 double bonds. In certain embodiments, R₂ contains 3 double bonds when the acyl chain length is over 19. In certain embodiments, R₂ contains 4 double bonds when the acyl chain length is over 21.

In certain embodiments, the length of the acyl chain of R₂ is different from the length of the acyl chain of R₃. In certain embodiments, the length of the acyl chain of R₂ is different from the length of the acyl chain of R₃ and R₂ is unsaturated (containing for example 1 to 4 double bonds) and R₃ is saturated (containing no double bonds). In certain embodiments, R₂ contains 3 double bonds when the acyl chain length is over 19 (containing from example from 3 to 4 double bonds) and R₃ is saturated. In certain embodiments, R₂ contains 4 double bonds when the acyl chain length is over 21 and R₃ is saturated.

R₃ is a saturated or unsaturated acyl chain from 3 to 25 carbons in length. In one embodiment, R₃ is an acyl chain from 3 to 5 carbons in length, an acyl chain from 6 to 10 carbons in length, an acyl chain from 10 to 20 carbons in length, or an acyl chain from 21 to 25 carbons in length. In a particular aspect, R₃ is an acyl chain from 10 to 20 carbons in length. In another particular aspect, R₃ is an acyl chain from 15 to 19 carbons in length. Such acyl chains, regardless of the length, include both even and odd chain lengths and may be saturated or unsaturated. In a particular embodiment, R₃ is an acyl chain having an odd number of carbon atoms. In another embodiment, R₃ is an acyl chain of 3, 4, 5, 6, 7, 8, 9, 10, 11, 12, 13, 14, 15, 16, 17, 18, 19, 20, 21, 22, 23, 24, 25, 26, 27, 28, 29, or 30 carbons, which are saturated or unsaturated. In another embodiment, R₃ is an acyl chain of 10, 11, 12, 13, 14, 15, 16, 17, 18, 19, 20, 21, or 22, carbons, which is saturated or unsaturated. In another embodiment, R₃ is an acyl chain of 14, 15, 16, 17, or 18 carbons, which are saturated or unsaturated. In a preferred embodiment, acyl chains of the foregoing lengths are saturated.

In one embodiment, R₃ is a saturated acyl chain. In another embodiment, R₃ is an unsaturated acyl chain. When R₃ is an unsaturated acyl chain, the acyl chain may contain from 1 to 4, such as from 1 to 2 double bonds. In one embodiment, R₃ is an unsaturated acyl chain, containing 1 double bond. The double bonds in such unsaturated acyl chains may be present in the cis or trans configuration, or a mixture of cis and trans configuration when more than 1 double bond is present.

In one embodiment, when multiple diacylglycerol species are present, the R₃ groups on the various species are selected to include both saturated and unsaturated acyl chains (for example, with 1 to 2 double bonds). In one embodiment, when multiple diacylglycerol species are present, the R₃ groups on the various species are selected to include only saturated acyl chains.

In certain embodiments, the length of the acyl chain of R₃ is different from the length of the acyl chain of R₂. In certain embodiments, the length of the acyl chain of R₃ is different from the length of the acyl chain of R₂ and R₃ is saturated and R₂ is unsaturated (containing for example 1 to 4 double bonds). In certain embodiments, R₂ contains 3 double bonds when the acyl chain length is over 19 and R₃ is saturated. In certain embodiments, R₂ contains 4 double bonds when the acyl chain length is over 21 and R₃ is saturated.

In one embodiment of any of the foregoing, the isotopically labeled H is deuterium (D). In one embodiment of any of the foregoing, at least 1, at least 2, at least 3, at least 4 or all 5 of R₄ are isotopically labeled H, preferably deuterium (D).

In certain embodiments, at least a subset of the diacylglycerol species included in the ULQS have the same R₃ groups and different R₂ groups. In certain embodiments, all of the diacylglycerol species included in the ULQS have the same R₃ groups and different R₂ groups, and the R₂ groups are unsaturated acyl chains (for example, with 1 to 4 double bonds). In certain embodiments, all of the diacylglycerol species included in the ULQS have the same R₃ groups and different R₂ groups, and the R₃ groups are saturated acyl chains, the R₂ groups are unsaturated acyl chains (for example, with 1 to 4 double bonds). In certain embodiments, all of the diacylglycerol species included in the ULQS have the same R₃ groups and different R₂ groups, and the R₂ groups are unsaturated acyl chains (for example, with 1 to 4 double bonds) and includes acyl chains with 2 to 4 double bonds. In certain embodiments, all of the diacylglycerol species included in the ULQS have the same R₃ groups and different R₂ groups, and the R₃ groups are saturated acyl chains, the R₂ groups are unsaturated acyl chains (for example, with 1 to 4 double bonds) and includes acyl chains with 3 to 4 double bonds. In certain embodiments of the foregoing, R₂ contains 3 double bonds when the acyl chain length is over 19 and 4 double bonds when the acyl chain length is over 21.

In one embodiment, the diacylglycerol species included in the ULQS include those species wherein R₃ is independently an acyl chain of n carbon atoms in length, n is 14 to 20, R₂ is independently an acyl chain from n-5 to n+7 carbon atoms, wherein R₃ optionally contains 1 or 2 double bonds (preferably 0 double bonds), R₂ contains 1 or 2 double bonds (preferably 1 double bond) when the length of R₂ is from n-5 to n-1 carbon atoms, and R₂ contains 2 to 4 double bonds (preferably 3 or 4 double bonds) when the length of R₂ is from n+2 to n+7 carbon atoms.

In one embodiment, the diacylglycerol species included in the ULQS include those species wherein R₃ is independently an acyl chain of n carbon atoms in length, n is 15, 17, or 19, R₂ is independently an acyl chain from n-5 to n+7 carbon atoms, wherein R₃ optionally contains 1 or 2 double bonds (preferably 0 double bonds), R₂ contains 1 or 2 double bonds (preferably 1 double bond) when the length of R₂ is from n-5 to n-1 carbon atoms, and R₂ contains 2 to 4 double bonds (preferably 3 or 4 double bonds) when the length of R₂ is from n+2 to n+7 carbon atoms.

In one embodiment, the diacylglycerol species included in the ULQS include those species wherein R₃ is independently a saturated acyl chain of n carbon atoms in length, n is 15, 17, or 19, R₂ is independently an acyl chain from n-5 to n+7 carbon atoms, wherein R₃ optionally contains 1 or 2 double bonds (preferably 0 double bonds), R₂ contains 1 or 2 double bonds (preferably 1 double bond) when the length of R₂ is from n-5 to n-1 carbon atoms, R₂ contains 2 to 3 double bonds (preferably 3 double bonds) when the length of R₂ is from n+2 to n+3 carbon atoms, and R₂ contains 3 to 4 double bonds (preferably 4 double bonds) when the length of R₂ is from n+4 to n+7 carbon atoms.

In one embodiment, the diacylglycerol species suitable for inclusion in the ULQS may be represented by the formula V:
wherein:
at least one of R₄ (preferably all of R₄) is deuterium;
R₃ is a saturated acyl chain of 15, 17, or 19 carbons in length; and
R₂ is an acyl chain of 12, 14, 16, 18, 20, 22 or 24 carbons in length, wherein R₂ contains 1 double bond when R₂ is 12, 14, 16, or 18 carbons in length, R₂ contains 3 double bonds when R₂ is 20 carbons in length, and R₂ contains 4 double bonds when R₂ is 22 or 24 carbons in length.

In a particular aspect of this embodiment, the diacylglycerol species included in the ULQS consists of 5 species wherein R₃ is a saturated acyl chain of 15 carbons in length and R₂ is 14:1, 16:1, 18:1, 20:3, and 22:4.

In another particular aspect of this embodiment, the diacylglycerol species included in the ULQS consists of 5 species wherein R₃ is a saturated acyl chain of 17 carbons in length and R₂ is 14:1, 16:1, 18:1, 20:3, and 22:4.

In another particular aspect of this embodiment, the diacylglycerol species included in the ULQS consists of 5 species wherein R₃ is a saturated acyl chain of 19 carbons in length and R₂ is 14:1, 16:1, 18:1, 20:3, and 22:4.

In a specific embodiment, suitable diacylglycerol species suitable for inclusion in the ULQS include those listed in Table 5 below (with reference to formula V, wherein at least one of R₄ (preferably all of R₄) is deuterium, the underlined portion corresponds to the R₂ variable and the double underlined portion correspond to the R₃ variable).

| Table 5 | | |
|---|---|---|
| DAG (17:0/14:1) | DAG (17:0/18.1) | DAG (17:0/22:4) |
| DAG (17:0/16:1) | DAG (17:0/20:3) | |

### Ceramides

In one embodiment, the ceramide class includes a plurality of ceramide species, wherein each ceramide species contains an isotopic label. Any combination of ceramide described herein may be used. In one embodiment, the acyl chain of the ceramide species are independently selected from saturated C10 to C30 acyl chain or an unsaturated C10 to C30 acyl chain, containing from 1 to 4 double bonds, such as from 1 to 2 double bonds or 1 double bond.

In one embodiment, the acyl chain of the ceramide species is a saturated or unsaturated C10 to C15 acyl chain, a saturated or unsaturated C16 to C20 acyl chain, a saturated or unsaturated C15 to C25 acyl chain, a saturated or unsaturated C21 to C25 acyl chain, a saturated or unsaturated C26 to C30 acyl chain. Such acyl chain, regardless of the length, includes both even and odd chain lengths and may be saturated or unsaturated. In a particular embodiment, the acyl chain of the ceramide species are selected such that the sum composition score for the acyl chain is an even number. In a particular embodiment, the acyl chain of the ceramide species are each independently an acyl chain of 10, 11, 12, 13, 14, 15, 16, 17, 18, 19, 20, 21, 22, 23, 24, 25, 26, 27, 28, 29, or 30 carbons, which are saturated or unsaturated (preferably unsaturated). In another particular embodiment, the acyl chain of the ceramide species are each independently an acyl chain of 10, 11, 12, 13, 14, 15, 16, 17, 18, 19, 20, 21, 22, 23, 24 or 25 carbons, which are saturated or unsaturated (preferably unsaturated). In still another particular embodiment, the acyl chain of the ceramide species are each independently an acyl chain of 16, 17, 18, 19, 20, 21, 22, 23, or 24 carbons, which are saturated or unsaturated (preferably unsaturated).

In one embodiment, the acyl chain of the ceramide species are saturated. In another embodiment, the acyl chain of the ceramide species are unsaturated. When the acyl chain of the ceramide species is unsaturated, the acyl chain may contain from 1 to 4 double bonds. In one embodiment, the acyl chain is unsaturated, containing from 1 or 2 double bonds. The double bonds in such unsaturated acyl chains may be present in the cis or trans configuration, or a mixture of cis and trans configuration when more than 1 double bond is present.

In one embodiment, when multiple species of ceramide are present, the acyl chain on the various species are selected to include both saturated and unsaturated acyl chains (for example, with 1 to 4 double bonds, preferably 1 to 2 double bonds). In one embodiment, when multiple species of a ceramide are present, the acyl chain on the various species are selected to include unsaturated acyl chains only (for example, with 1 to 4 double bonds, preferably 1 to 2 double bonds).

In certain embodiments, when multiple species of ceramide are present, the sum composition scores of the acyl chain of the ceramide species are different. In certain embodiments, when multiple species of ceramide are present, the sum composition scores the acyl chain of the ceramide species are different and the acyl chains are each unsaturated (containing for example 1 to 4 double bonds, preferably 1 to 2 double bonds). In certain embodiments, when multiple species of ceramide are present, the sum composition scores the acyl chain of the ceramide species are different, the acyl chains are each unsaturated (containing for example 1 to 4 double bonds, preferably 1 to 2 double bonds), and the sum composition score for the acyl chain is an even number.

In any of the foregoing embodiments, the ceramide contains at least one isotopically labeled H atom, preferably from 2 to 7 isotopically labeled H atoms. In preferred embodiments, the isotopically labeled H atom is deuterium (D).

In one embodiment of the foregoing, the ceramides for inclusion in the ULQS are represented by the general formula VI, or a pharmaceutically acceptable salt thereof: 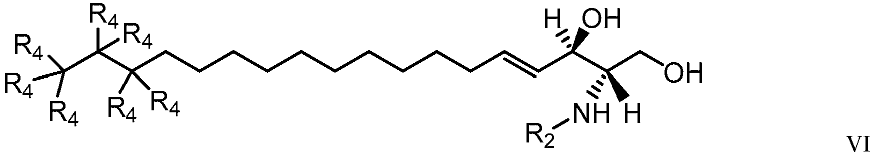 wherein:
R₂ is a C10 to C30 saturated or unsaturated acyl chain; and
R₄ is independently H or an isotope of H, provided that at least one of R₄ is an isotope of H.

R₂ is a saturated or unsaturated acyl chain from 10 to 30 carbons in length. In one embodiment, R₂ is an acyl chain from 10 to 15 carbons in length, an acyl chain from 16 to 20 carbons in length, an acyl chain from 21 to 25 carbons in length, or an acyl chain from 26 to 30 carbons in length. In a particular aspect, R₂ is an acyl chain from 10 to 25 carbons in length. In another particular aspect, R₂ is an acyl chain from 16 to 24. Such acyl chains, regardless of the length, include both even and odd chain lengths and may be saturated or unsaturated. In a particular embodiment, R₂ is an acyl chain having an even number of carbon atoms. In another embodiment, R₂ is an acyl chain of 10, 11, 12, 13, 14, 15, 16, 17, 18, 19, 20, 21, 22, 23, 24, 25, 26, 27, 28, 29, or 30 carbons, which are saturated or unsaturated (preferably unsaturated). In another embodiment, R₂ is an acyl chain of 10, 11, 12, 13, 14, 15, 16, 17, 18, 19, 20, 21, 22, 23, 24, or 25 carbons, which is saturated or unsaturated. In another embodiment, R₂ is an acyl chain of 16, 17, 18, 19, 20, 21, 22, 23, or 24 carbons, which are saturated or unsaturated (preferably unsaturated). In a preferred embodiment, acyl chains of the foregoing lengths are unsaturated containing from 1 to 4 double bonds, preferably 1 to 2 double bonds.

In one embodiment, R₂ is a saturated acyl chain. In another embodiment, R₂ is an unsaturated acyl chain. When R₂ is an unsaturated acyl chain, the acyl chain may contain from 1 to 4 double bonds. In one embodiment, R₂ is an unsaturated acyl chain, containing from 1 or 2 double bonds. The double bonds in such unsaturated acyl chains may be present in the cis or trans configuration, or a mixture of cis and trans configuration when more than 1 double bond is present.

In one embodiment, when multiple ceramide species are present, the R₂ group on the various species are selected to include both saturated and unsaturated acyl chains (for example, with 1 to 4 double bonds). In one embodiment, when multiple ceramide species, the R₂ group on the various species are selected to include only unsaturated acyl chains (for example, with 1 to 4 double bonds, preferably 1 to 2 double bonds).

In one embodiment of any of the foregoing, the isotopically labeled H is deuterium (D). In one embodiment of any of the foregoing, at least 1, at least 2, at least 3, at least 4, at least 5, at least 6 or all 7 of R₄ are isotopically labeled H, preferably deuterium (D).

In certain embodiments, at least a subset of the ceramide species included in the ULQS have different R₂ groups. In certain embodiments, all of the ceramide species included in the ULQS have different R₂ groups and the R₂ groups are each unsaturated (for example, with 1 to 2 double bonds).

In one embodiment, the ceramide species suitable for inclusion in the ULQS may be represented by the formula VI:
wherein:
at least one of R₄ (preferably all of R₄) is deuterium; and
R₂ is an acyl chain of 12, 14, 16, 18, 20, 22, 24, 26, or 28 carbons in length, wherein R₂ contains 1 double bond.

In a particular aspect of this embodiment, the ceramide species suitable for inclusion in the ULQS consists of 5 species wherein R₂ is 12:1, 14:1, 16:1, 18:1, and 20:1.

In a particular aspect of this embodiment, the ceramide species suitable for inclusion in the ULQS consists of 5 species wherein R₂ is 14:1, 16:1, 18:1, 20:1, and 22:1.

In a particular aspect of this embodiment, the ceramide species suitable for inclusion in the ULQS consists of 5 species wherein R₂ is 16:1, 18:1, 20:1, 22:1, and 24:1.

In a particular aspect of this embodiment, the ceramide species suitable for inclusion in the ULQS consists of 5 species wherein R₂ is 18:1, 20:1, 22:1, 24:1, and 26:1.

In a particular aspect of this embodiment, the ceramide species suitable for inclusion in the ULQS consists of 5 species wherein R₂ is 20:1, 22:1, 24:1, 26:1, and 28:1.

In a specific embodiment, suitable ceramide species suitable for inclusion in the ULQS include those listed in Table 6 below (with reference to formula VI, wherein at least one of R₄ (preferably all of R₄) is deuterium and the underlined portion corresponds to the R₂ variable).

| Table 6 | | |
|---|---|---|
| CER (18:1/16:1) | CER (18:1/20:1) | CER (18:1/24:1) |
| CER (18:1/18:1) | CER (18:1/22:1) | |

### Sphingomyelin

In one embodiment, the sphingomyelin class includes a plurality of sphingomyelin species. Any combination of sphingomyelin species described herein may be used. In one embodiment, the acyl chain of the sphingomyelin species are independently selected from saturated C10 to C30 acyl chains or unsaturated C10 to C30 acyl chains containing from 1 to 4 double bonds, such as from 1 to 2 double bonds or 1 double bond. In one embodiment, the sphingomyelin species comprise a phosphatidylcholine group. In another embodiment, the sphingomyelin species comprise a phosphatidylethanolamine group.

In one embodiment, the acyl chain of the sphingomyelin species is independently a saturated or unsaturated C10 to C15 acyl chain, a saturated or unsaturated C16 to C20 acyl chain, a saturated or unsaturated C21 to C25 acyl chain, or a saturated or unsaturated C26 to C30 acyl chain. Such acyl chains, regardless of the length, includes both even and odd chain lengths and may be saturated or unsaturated. In a particular embodiment, the acyl chain of the sphingomyelin species are each selected such that the sum composition score is an even number. In a particular embodiment, the acyl chain of the sphingomyelin species are independently an acyl chain of 10, 11, 12, 13, 14, 15, 16, 17, 18, 19, 20, 21, 22, 23, 24, 25, 26, 27, 28, 29, or 30 carbons, which are saturated or unsaturated (preferably unsaturated). In another particular embodiment, the acyl chain of the sphingomyelin species are independently an acyl chain of 10, 11, 12, 13, 14, 15, 16, 17, 18, 19, 20, 21, 22, 23, 24 or 25 carbons, which are saturated or unsaturated (preferably unsaturated). In still another particular embodiment, the acyl chain of the sphingomyelin species are independently an acyl chain of 16, 17, 18, 19, 20, 21, 22, 23, or 24 carbons, which are saturated or unsaturated (preferably unsaturated).

In one embodiment, the acyl chain of the sphingomyelin species is saturated. In another embodiment, the acyl chain of the sphingomyelin species is unsaturated. When one or more of the acyl chain of the sphingomyelin species is unsaturated, the acyl chain may contain from 1 to 4 double bonds, preferably 1 to 2 double bonds. In one embodiment, the acyl chain is unsaturated, containing from 1 or 2 double bonds, preferably 1 double bond. The double bonds in such unsaturated acyl chains may be present in the cis or trans configuration, or a mixture of cis and trans configuration when more than 1 double bond is present.

In one embodiment, when multiple sphingomyelin species are present, the acyl chain on the various species is selected to include both saturated and unsaturated acyl chains (for example, with 1 to 4 double bonds). In one embodiment, when multiple sphingomyelin species are present, the acyl chain on the various species is selected to include unsaturated acyl chains only (for example, with 1 to 4 double bonds, preferably 1 to 2 double bonds, more preferably 1 double bond).

In certain embodiments, when multiple sphingomyelin species are present, the sum composition scores of the different species are different from one another. In certain embodiments, when multiple sphingomyelin species are present, the sum composition scores of the different species are different from one another and the acyl chain are each unsaturated (containing for example 1 to 4 double bonds, preferably from 1 to 2 double bonds, more preferably 1 double bond) and the sum composition score for the acyl chains are an even number.

In any of the foregoing embodiments, the sphingomyelin contains at least one isotopically labeled H atom, preferably from 2 to 9 isotopically labeled H atoms. In preferred embodiments, the isotopically labeled H atom is deuterium (D).

In one embodiment of the foregoing, the sphingomyelins for inclusion in the ULQS are represented by the general formula VII: 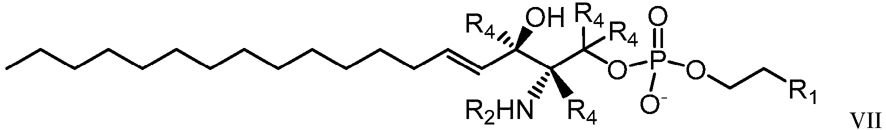 wherein:
R₁ is 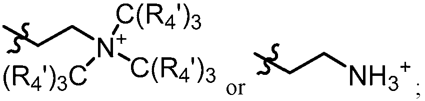
R₂ is a C10 to C30 saturated or unsaturated acyl chain; and
R₄ and R₄' are each independently H or an isotope of H, provided that at least one of R₄ or R₄' is an isotope of H.

In one embodiment, R₁ is 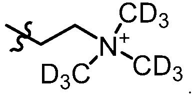 In one embodiment, when R₁ is 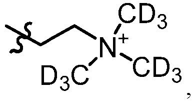 then R₄ are each H. In another embodiment R₁ is 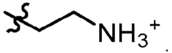 When R₁ is 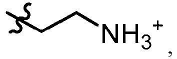 the at least one of R₄, preferably all of R₄, is an isotope of H (preferably deuterium, D).

R₂ is a saturated or unsaturated acyl chain from 10 to 30 carbons in length. In one embodiment, R₂ is an acyl chain from 10 to 15 carbons in length, an acyl chain from 16 to 20 carbons in length, an acyl chain from 21 to 25 carbons in length, or an acyl chain from 26 to 30 carbons in length. In a particular aspect, R₂ is an acyl chain from 10 to 25 carbons in length. In another particular aspect, R₂ is an acyl chain from 16 to 24. Such acyl chains, regardless of the length, include both even and odd chain lengths and may be saturated or unsaturated. In a particular embodiment, R₂ is an acyl chain having an even number of carbon atoms. In another embodiment, R₂ is independently an acyl chain of 10, 11, 12, 13, 14, 15, 16, 17, 18, 19, 20, 21, 22, 23, 24, 25, 26, 27, 28, 29, or 30 carbons, which are saturated or unsaturated (preferably unsaturated). In another embodiment, R₂ is independently an acyl chain of 10, 11, 12, 13, 14, 15, 16, 17, 18, 19, 20, 21, 22, 23, 24, or 25 carbons, which is saturated or unsaturated. In another embodiment, R₂ is independently an acyl chain of 16, 17, 18, 19, 20, 21, 22, 23, or 24 carbons, which are saturated or unsaturated (preferably unsaturated). In a preferred embodiment, acyl chains of the foregoing lengths are unsaturated containing from 1 to 4 double bonds.

In one embodiment, R₂ is a saturated acyl chain. In another embodiment, R₂ is an unsaturated acyl chain. When R₂ is an unsaturated acyl chain, the acyl chain may contain from 1 to 4 double bonds. In one embodiment, R₂ is an unsaturated acyl chain, containing from 1 or 2 double bonds. The double bonds in such unsaturated acyl chains may be present in the cis or trans configuration, or a mixture of cis and trans configuration when more than 1 double bond is present.

In one embodiment, when multiple sphingomyelin species are present, the R₂ group on the various species are selected to include both saturated and unsaturated acyl chains (for example, with 1 to 4 double bonds). In one embodiment, when multiple sphingomyelin species are present, the R₂ group on the various species are selected to include only unsaturated acyl chains (for example, with 1 to 4 double bonds).

In one embodiment of any of the foregoing, the isotopically labeled H is deuterium (D). In one embodiment of any of the foregoing, at least 1, at least 2, at least 3, or all 4 of R₄ are isotopically labeled H, preferably deuterium (D). In one embodiment of any of the foregoing, at least 1, at least 2, at least 3, at least 4, at least 5, at least 6, at least 7, at least 8, or all 9 of R₄' are isotopically labeled H, preferably deuterium (D). In one embodiment of any of the foregoing, when at least 1, at least 2, at least 3, at least 4, at least 5, at least 6, at least 7, at least 8, or all 9 of R₄' are isotopically labeled H, preferably deuterium (D), then at least one, at least two, at least 3, or preferably all 4 of R₄ are H.

In certain embodiments, at least a subset of the sphingomyelin species included in the ULQS have the same R₁ group and different R₂ groups. In certain embodiments, at least a subset of the sphingomyelin species included in the ULQS have the same R₁ group, different R₂ groups and the R₂ groups are each unsaturated (for example, with 1 to 2 double bonds).

In one embodiment, the sphingomyelin species suitable for inclusion in the ULQS may be represented by the formulas VIIB below: 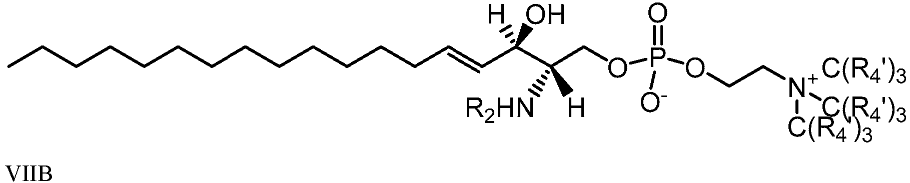 wherein:
at least one of R₄' (preferably all of R₄') is deuterium; and
R₂ is an acyl chain of 12, 14, 16, 18, 20, 22, 24, 26, or 28 carbons in length, wherein R₂ contains 1 double bond.

In a particular aspect of this embodiment, the sphingomyelin species suitable for inclusion in the ULQS consists of 5 species wherein R₂ is 12:1, 14:1, 16:1, 18:1, and 20:1.

In a particular aspect of this embodiment, the sphingomyelin species suitable for inclusion in the ULQS consists of 5 species wherein R₂ is 14:1, 16:1, 18:1, 20:1, and 22:1.

In a particular aspect of this embodiment, the sphingomyelin species suitable for inclusion in the ULQS consists of 5 species wherein R₂ is 16:1, 18:1, 20:1, 22:1, and 24:1.

In a particular aspect of this embodiment, the sphingomyelin species suitable for inclusion in the ULQS consists of 5 species wherein R₂ is 18:1, 20:1, 22:1, 24:1, and 26:1.

In a particular aspect of this embodiment, the sphingomyelin species suitable for inclusion in the ULQS consists of 5 species wherein R₂ is 20:1, 22:1, 24:1, 26:1, and 28:1.

In one embodiment, the sphingomyelin species suitable for inclusion in the ULQS may be represented by the formulas VIIC below: 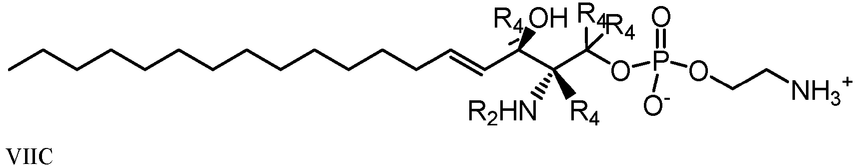 wherein:
at least one of R₄ (preferably all of R₄) is deuterium; and
R₂ is an acyl chain of 12, 14, 16, 18, 20, 22, 24, 26, or 28 carbons in length, wherein R₂ contains 1 double bond.

In a particular aspect of this embodiment, the sphingomyelin species suitable for inclusion in the ULQS consists of 5 species wherein R₂ is 12:1, 14:1, 16:1, 18:1, and 20:1.

In a particular aspect of this embodiment, the sphingomyelin species suitable for inclusion in the ULQS consists of 5 species wherein R₂ is 14:1, 16:1, 18:1, 20:1, and 22:1.

In a particular aspect of this embodiment, the sphingomyelin species suitable for inclusion in the ULQS consists of 5 species wherein R₂ is 16:1, 18:1, 20:1, 22:1, and 24:1.

In a particular aspect of this embodiment, the sphingomyelin species suitable for inclusion in the ULQS consists of 5 species wherein R₂ is 18:1, 20:1, 22:1, 24:1, and 26:1.

In a particular aspect of this embodiment, the sphingomyelin species suitable for inclusion in the ULQS consists of 5 species wherein R₂ is 20:1, 22:1, 24:1, 26:1, and 28:1.

In a specific embodiment, sphingomyelin species included in the ULQS include those listed in Table 7 below (with reference to formula VIIB), wherein at least one of R₄' (preferably all of R₄') is deuterium and the underlined portion corresponds to the R₂ variable.

| Table 7 | | |
|---|---|---|
| SM (18:1/16:1) | SM (18:1/20:1) | SM (18:1/24:1) |
| SM (18:1/18:1) | SM (18:1/22:1) | |

### Exemplary Combinations

As discussed herein, the ULQS may contain one or more lipid species from a one or more of the lipid classes described herein. It is understood that the selection of the classes of lipids for inclusion in the ULQS, the selection of the individual lipid species within each lipid class for inclusion in the ULQS, and the concentration of the individual lipid species within the ULQS may depend on a variety of factors, such as, but not limited to, the sample type to be analyzed or a specific research objective (for example, identifying lipid biomarkers for heart disease). In one embodiment, the ULQS contains one or more lipid species from at least three lipid classes. In one embodiment, the ULQS contains one or more lipid species from at least three lipid classes, wherein the classes of lipids are selected from i) phospholipids; ii) lysophospholipids; iii) cholesterol esters; iv) triacylglycerols; v) diacylglycerols; vi) ceramides; and vii) sphingomyelins).

In a first combination, the ULQS contains at least one lipid species from each of the following lipid classes: i) phospholipids; ii) lysophospholipids; iii) cholesterol esters; iv) triacylglycerols; v) diacylglycerols; vi) ceramides; and vii) sphingomyelins.

In a second combination, the ULQS contains at two or more lipid species from each of the following lipid classes: i) phospholipids; ii) lysophospholipids; iii) cholesterol esters; iv) triacylglycerols; v) diacylglycerols; vi) ceramides; and vii) sphingomyelins.

In a third combination, the ULQS contains each of the following lipid classes with number of lipid species in each lipid class indicated in parentheses: i) phospholipids (5 to 40); ii) lysophospholipids (5 to 25); iii) cholesterol esters (3 to 15); iv) triacylglycerols (5 to 20); v) diacylglycerols (3 to 15); vi) ceramides (3 to 15); and vii) sphingomyelins (3 to 15).

In a fourth combination, the ULQS contains each of the following lipid classes with number of lipid species in each lipid class indicated in parentheses: i) phospholipids (5 to 40); ii) lysophospholipids (5 to 25); iii) cholesterol esters (3 to 15); iv) triacylglycerols (5 to 20); v) diacylglycerols (3 to 15); vi) ceramides (3 to 15); and vii) sphingomyelins (3 to 15) and the lipid species and concentration of the lipid species are selected from Table 8 below.

In a fifth combination, the ULQS contains each of the following lipid classes with number of lipid species in each lipid class indicated in parentheses: i) phospholipids (25); ii) lysophospholipids (15); iii) cholesterol esters (5); iv) triacylglycerols (9); v) diacylglycerols (5); vi) ceramides (5); and vii) sphingomyelins (5) and the lipid species and concentration of the lipid species are selected from Table 8 below.

| **Table 8** | | |
|---|---|---|
| **Internal Standard Component** | **Formula** | **Conc µg/mL** |
| 14:0-13:0-14:0 TG-d5 | C₄₄H₇₉D₅O₆ | 25 |
| 14:0-15:1-14:0 TG-d5 | C₄₆H₈₁D₅O₆ | 50 |
| 14:0-17:1-14:0 TG-d5 | C₄₈H₈₅D₅O₆ | 75 |
| 16:0-15:1-16:0 TG-d5 | C₅₀H₈₉D₅O₆ | 100 |
| 16:0-17:1-16:0 TG-d5 | C₅₂H₉₃D₅O₆ | 125 |
| 16:0-19:2-16:0 TG-d5 | C₅₄H₉₅D₅O₆ | 100 |
| 18:1-17:1-18:1 TG-d5 | C₅₆H₉₇D₅O₆ | 75 |
| 18:1-19:2-18:1 TG-d5 | C₅₈H₉₉D₅O₆ | 50 |
| 18:1-21:2-18:1 TG-d5 | C₆₀H₁₀₃D₅O₆ | 25 |
| | | |
| 14:1 cholesteryl-d7 ester | C₄₁H₆₃D₇O₂ | 25 |
| 16:1 cholesteryl-d7 ester | C₄₃H₆₇D₇O₂ | 50 |
| 18:1 cholesteryl-d7 ester | C₄₃H₇₁D₇O₂ | 75 |
| 20:3 cholesteryl-d7 ester | C₄₇H₇₁D₇O₂ | 50 |
| 22:4 cholesteryl-d7 ester | C₄₉H₇₃D₇O₂ | 25 |
| | | |
| C16:1 Ceramide-d7 | C₃₄H₅₈D₇NO₃ | 75 |
| C18:1 Ceramide-d7 | C₃₆H₆₂D₇NO₃ | 50 |
| C20:1 Ceramide-d7 | C₃₈H₆₆D₇NO₃ | 25 |
| C22:1 Ceramide-d7 | C₄₀H₇₀D₇NO₃ | 50 |
| C24:1 Ceramide-d7 | C₄₂H₇₄D₇NO₃ | 75 |
| | | |
| 16:1 SM (d18:1/16:1)-d9 | C₃₉H₆₈D₉N₂O₆P | 75 |
| 18:1 SM (d18:1/18:1)-d9 | C₄₁H₇₂D₉N₂O₆P | 50 |
| 20:1 SM (d18:1/20:1)-d9 | C₄₃H₇₆D₉N₂O₆P | 25 |
| 22:1 SM (d18:1/22:1)-d9 | C₄₅H₈₀D₉N₂O₆P | 50 |
| 24:1 SM (d18:1/24:1)-d9 | C₄₇H₈₄D₉N₂O₆P | 75 |
| | | |
| 17:0-14:1 PC-d5 | C₃₉H₇₁D₅NO₈P | 50 |
| 17:0-16:1 PC-d5 | C₄₁H₇₅D₅NO₈P | 100 |
| 17:0-18:1 PC-d5 | C₄₃H₇₉D₅NO₈P | 150 |
| 17:0-20:3 PC-d5 | C₄₅H₇₉D₅NO₈P | 100 |
| 17:0-22:4 PC-d5 | C₄₇H₈₁D₅NO₈P | 50 |
| 17:0-14:1 PE-d5 | C₃₆H₆₅D₅NO₈P | 25 |
| 17:0-16:1 PE-d5 | C₃₈H₆₉D₅NO₈P | 50 |
| 17:0-18:1 PE-d5 | C₄₀H₇₃D₃NO₈P | 75 |
| 17:0-20:3 PE-d5 | C₄₂H₇₃D₅NO₈P | 50 |
| 17:0-22:4 PE-d5 | C₄₄H₇₅D₅NO₈P | 25 |
| 17:0-14:1 PG-d5 | C₃₇H₆₅D₅NaO₁₀P | 25 |
| 17:0-16:1 PG-d5 | C₃₉H₆₉D₅NaO₁₀P | 50 |
| 17:0-18:1 PG-d5 | C₄₁H₇₃D₅NaO₁₀P | 75 |
| 17:0-20:3 PG-d5 | C₄₃H₇₃D₃NaO₁₀P | 50 |
| 17:0-22:4 PG-d5 | C₄₃H₇₃D₃NaO₁₀P | 25 |
| 17:0-14:1 PS-d5 | C₃₇H₆₄D₅NNaO₁₀P | 25 |
| 17:0-16:1 PS-d5 | C₃₉H₆₈D₅NNaO₁₀P | 50 |
| 17:0-18:1 PS-d5 | C₄₁H₇₂D₅NNaO₁₀P | 75 |
| 17:0-20:3 PS-d5 | C₄₃H₇₂D₅NNaO₁₀P | 50 |
| 17:0-22:4 PS-d5 | C₄₃H₇₄D₃NNaO₁₀P | 25 |
| 17:0-14:1 PI-d5 | C₄₀H₇₃D₃NO₁₃P | 25 |
| 17:0-16:1 PI-d5 | C₄₂H₇₇D₅NO₁₃P | 50 |
| 17:0-18:1 PI-d5 | C₄₄H₈₁D₅NO₁₃P | 75 |
| 17:0-20:3 PI-d5 | C₄₆H₈₁D₃NO₁₃P | 50 |
| 17:0-22:4 PI-d5 | C₄₈H₈₃D₅NO₁₃P | 25 |
| | | |
| 17:0-14:1 DG-d5 | C₃₄H₅₉D₅O₅ | 25 |
| 17:0-16:1 DG-d5 | C₃₆H₆₃D₅O₅ | 50 |
| 17:0-18:1 DG-d5 | C₃₈H₆₇D₅O₅ | 75 |
| 17:0-20:3 DG-d5 | C₄₀H₆₇D₅O₅ | 50 |
| 17:0-22:4 DG-d5 | C₄₂H₆₉D₅O₅ | 25 |
| | | |
| 15:0 Lyso PI-d5 | C₂₄H₄₅D₅NO₁₂P | 25 |
| 17:0 Lyso PI-d5 | C₂₆H₄₉D₅NO₁₂P | 50 |
| 19:0 Lyso PI-d5 | C₂₈H₅₃D₅NO₁₂P | 25 |
| 15:0 Lyso PS-d5 | C₂₁H₃₆D₅NNaO₉P | 25 |
| 17:0 Lyso PS-d5 | C₂₃H₄₀D₅NNaO₉P | 50 |
| 19:0 Lyso PS-d5 | C₂₅H₄₄D₃NNaO₉P | 25 |
| 15:0 Lyso PG-d5 | C₂₁H₃₇D₅NaO₉P | 25 |
| 17:0 Lyso PG-d5 | C₂₃H₄₁D₅NaO₉P | 50 |
| 19:0 Lyso PG-d5 | C₂₃H₄₃D₃NaO₉P | 25 |
| 15:0 Lyso PC-d5 | C₂₃H₄₃D₃NO₇P | 25 |
| 17:0 Lyso PC-d5 | C₂₅H₄₇D₅NO₇P | 50 |
| 19:0 Lyso PC-d5 | C₂₇H₅₁D₅NO₇P | 25 |
| 15:0 Lyso PE-d5 | C₂₀H₃₇D₃NO₇P | 25 |
| 17:0 Lyso PE-d5 | C₂₂H₄₁D₃NO₇P | 50 |
| 19:0 Lyso PE-d5 | C₂₄H₄₅D₅NO₇P | 25 |

In an aspect of any of the first to third combinations, the members of each lipid class are selected from the lipids species disclosed in Formulas I to VII herein, optionally with additional lipid species, and the concentrations of the various lipid species is independently selected from 10 to 500 µg/ml as described herein.

In an aspect of any of the first to third combinations, the members of each lipid class are selected from the lipids species disclosed in Tables 1 to 7 herein, optionally with additional lipid species, and the concentrations of the various lipid species is independently selected from 10 to 500 µg/ml as described herein.

For any of the above phospholipid species, at least one of R₄ (preferably all of R₄) is deuterium and/or the sum composition score for the lipid species are as follows: i) phospholipids- 27:1 to 43:4 (preferably 31:1 to 39:4); ii) lysophospholipids- 11:0 to 23:0 (preferably 15:0 to 19:0); iii) cholesterol esters- 11:1 to 25:4 (preferably 14:1 to 22:4); iv) triacylglycerols- 43:1 to 55:2 (preferably 47:1 to 51:2); v) diacylglycerols- 27:1 to 43:4 (preferably 31:1 to 39:4); vi) ceramides- 12:1 to 28:1 (preferably 16:1 to 24:1); and vii) sphingomyelins- 12:1 to 28:1 (preferably 16:1 to 24:1).

In one embodiment of any of the above, the selection of the individual species for inclusion in the ULQS are selected to correct for, among other things, at least one of the following: ionization efficiency, extraction efficiency, and differential fragmentation efficiency of at least one lipid species in the sample to be analyzed or tested. In certain embodiments, the individual lipid species selected for inclusion in the ULQS are selected to correct for differential fragmentation efficiency and at least one of ionization efficiency and extraction efficiency (preferably both ionization efficiency and extraction efficiency). In certain embodiments, the individual lipid species selected for inclusion in the ULQS. In certain embodiments, the individual lipid species selected for inclusion in the ULQS are selected to correct for at least one of the following: ionization efficiency, extraction efficiency, and differential fragmentation efficiency of at least one lipid species in the sample to be tested and the various lipid species for inclusion in the ULQS from each class are further selected to: i) cover the entire mass range or substantially the entire mass range of at least one lipid species present in the sample; ii) to mirror the concentration of at least one lipid species present in the sample; or iii) cover the entire mass range or substantially the entire mass range of at least one lipid species present in the sample and to mirror the concentration of at least one lipid species present in the sample.

In certain embodiments, the individual lipid species selected for inclusion in the ULQS are selected to correct for differential fragmentation efficiency of at least one lipid species in the sample to be analyzed or tested and at least one of ionization efficiency and extraction efficiency (preferably both ionization efficiency and extraction efficiency) of at least one lipid species in the sample to be analyzed/tested and the various lipid species for inclusion in the ULQS from each class are further selected to: i) cover the entire mass range or substantially the entire mass range of at least one lipid species present in the sample; ii) to mirror the concentration of at least one lipid species present in the sample; or iii) cover the entire mass range or substantially the entire mass range of at least one lipid species present in the sample and to mirror the concentration of at least one lipid species present in the sample.

### PACKAGING

The ULQS of the present disclosure may be provided in one or more container. It is preferred that the interior surfaces of the container are not reactive with the components, including the lipid components, of the ULQS. An exemplary container suitable for use for use with the ULQS is available from Wheaton.

The ULQS may be provided dispersed/dissolved in one or more solvents or as a powder (for example, a lyophilized powder). Suitable solvents include, but are not limited to, methanol, and dichloromethane/methanol (1: 1). Other solvents may be used as is known in the art. The individual lipid species in the ULQS, whether contained in a single container or more than one container, may be present at the same concentration. Alternatively, one or more of the individual lipid species in the ULQS, whether contained in a single container or more than one container, may be present at a different concentration. Any suitable concentration for the individual lipid species may be used. In one embodiment, the individual lipid species are present from 10 to 500 µg/ml in a 1 ml volume when dispersed/dissolved in a solvent or at from 10 to 500 µg when provided as a powder.

The individual lipid species for inclusion in the ULQS and the concentration of the individual lipid species within the ULQS may depend on a variety of factors, such as, but not limited to, the sample type to be analyzed or a specific research objective (for example, identifying lipid biomarkers for heart disease). For example, the individual lipid species and the concentration of the individual lipid species may be optimized for analysis of plasma samples. For example, when the ULQS is optimized for plasma sample, the ULQS may comprise PC, PE, PS, PG, and PI and the concentration of the 5 species be: PC>PG>PI>PE>PS or PC>PG=PI=PE=PS.

In one embodiment, the ULQS is provide in a single container and the individual lipid species of the ULQS are all contained in the single container. The individual lipid species may be present at the same concentration or at different concentrations.

In another embodiment, the ULQS is provide in more than 1 container and the individual lipid species of the ULQS are all contained at least two containers. In one aspect of this embodiment, species from each individual class of lipid compounds (for example, i) phospholipids; ii) lysophospholipids; iii) cholesterol esters; iv) triacylglycerols; v) diacylglycerols; vi) ceramides; and vii) sphingomyelins) is provided in a separate container. The individual lipid species within each class of lipid compounds may be present at the same concentration or at different concentrations. This approach allows for the ULQS to be customized as the concentration of the lipid species within each class may be determined by the user based on the factors discussed herein.

In another aspect of this embodiment, at least one individual class of lipid compounds (for example, i) phospholipids; ii) lysophospholipids; iii) cholesterol esters; iv) triacylglycerols; v) diacylglycerols; vi) ceramides; and vii) sphingomyelins) is provided in a separate container and/or at least one lipid species of the above lipid classes is provided in a separate container. For example, the lysophospholipid, cholesterol ester, triacylglycerol, diacylglycerol, ceramide, and sphingomyelin classes may each be provided in a separate container and the individual lipid species of the phospholipid class may each be provide in a separate container. The individual lipid species within each class of lipid compounds may be present at the same concentration or at different concentrations. This approach allows for the ULQS to be customized further as the concentration of the lipid species within each class and the concentration of each lipid species of the phospholipid class may be determined by the user based on the factors discussed herein.

In another aspect of this embodiment, all of the individual lipid species of each class of lipid compounds (for example, i) phospholipids; ii) lysophospholipids; iii) cholesterol esters; iv) triacylglycerols; v) diacylglycerols; vi) ceramides; and vii) sphingomyelins) are provided in a separate container. The individual lipid species may be present at the same concentration or at different concentrations. This approach allows for the ULQS to be customized even further as the concentration of the lipid species may be determined by the user based on the factors discussed herein.

In a preferred embodiment, the ULQS contains at least one lipid species from three or more of the following lipid classes: i) phospholipids; ii) lysophospholipids; iii) cholesterol esters; iv) triacylglycerols; v) diacylglycerols; vi) ceramides; and vii) sphingomyelins, and each lipid class is packaged in a separate container. In a particular aspect of this embodiment, the members of each lipid class are selected from the lipids species disclosed in Tables 1 to 7 herein or selected from the lipid species described by formulas I-VII, optionally with additional lipid species, at a concentration of 10 t0 500 µg/ml as described herein.

In another preferred embodiment, the ULQS contains at two or more lipid species from three or more of the following lipid classes: i) phospholipids; ii) lysophospholipids; iii) cholesterol esters; iv) triacylglycerols; v) diacylglycerols; vi) ceramides; and vii) sphingomyelins, and each lipid class is packaged in a separate container. In a particular aspect of this embodiment, the members of each lipid class are selected from the lipids species disclosed in Tables 1 to 7 herein or selected from the lipid species described by formulas I-VII, optionally with additional lipid species, at a concentration of 10 t0 500 µg/ml as described herein.

In another preferred embodiment, the ULQS contains at least one lipid species from each of the following lipid classes: i) phospholipids; ii) lysophospholipids; iii) cholesterol esters; iv) triacylglycerols; v) diacylglycerols; vi) ceramides; and vii) sphingomyelins, and each lipid class is packaged in a separate container. In a particular aspect of this embodiment, the members of each lipid class are selected from the lipids species disclosed in Tables 1 to 7 herein or selected from the lipid species described by formulas I-VII, optionally with additional lipid species, at a concentration of 10 t0 500 µg/ml as described herein.

In another preferred embodiment, the ULQS contains at two or more lipid species from each of the following lipid classes: i) phospholipids; ii) lysophospholipids; iii) cholesterol esters; iv) triacylglycerols; v) diacylglycerols; vi) ceramides; and vii) sphingomyelins, and each lipid class is packaged in a separate container. In a particular aspect of this embodiment, the members of each lipid class are selected from the lipids species disclosed in Tables 1 to 7 herein or selected from the lipid species described by formulas I-VII, optionally with additional lipid species, at a concentration of 10 t0 500 µg/ml as described herein.

In another preferred embodiment, the ULQS contains each of the following lipid classes with number of lipid species in each lipid class indicated in parentheses: i) phospholipids (5 to 40); ii) lysophospholipids (5 to 25); iii) cholesterol esters (3 to 15); iv) triacylglycerols (5 to 20); v) diacylglycerols (3 to 15); vi) ceramides (3 to 15); and vii) sphingomyelins (3 to 15), each lipid class is packaged in a separate container, and the lipid species and concentration of the lipid species are selected from Table 8 herein, optionally with additional lipid species.

In another preferred embodiment, the ULQS contains each of the following lipid classes with number of lipid species in each lipid class indicated in parentheses: i) phospholipids (25); ii) lysophospholipids (15); iii) cholesterol esters (5); iv) triacylglycerols (9); v) diacylglycerols (5); vi) ceramides (5); and vii) sphingomyelins (5), each lipid class is packaged in a separate container, and the lipid species and concentration of the lipid species are selected from Table 8 herein, optionally with additional lipid species.

### KITS

The present disclosure also provides for a kit for use in quantifying a lipid analyte by mass spectrometry. Suitable kits comprise a ULQS of the present disclosure, and optionally may include packaging material and instructions for use of the ULQS. In certain embodiments, the ULQS is provided as a packaged set of reagents containing defined amounts of lipid species, with each class of lipid being contained in a separate container as described herein, in amounts sufficient for at least one assay.

### EXAMPLES

### Example 1

As discussed herein, the ULQS may be used in any MS application. A typical MS experiment utilizing the ULQS is exemplified below. In this example, HILIC is exemplified as a fractionation technique. HILIC is a variant of normal phase liquid chromatography that partly overlaps with other chromatographic applications such as ion chromatography and reversed phase liquid chromatography. HILIC uses hydrophilic stationary phases with reversed-phase type eluents. Any polar chromatographic surface can be used for HILIC separations, including, but not limited to, simple unbonded silica, silanol, or diol bonded phases, amino or anionic bonded phases, amide bonded phases, cationic bonded phases, and zwitterionic bonded phases. A typical mobile phase for HILIC chromatography includes acetonitrile (MeCN) with a small amount of water. However, any aprotic solvent miscible with water (e.g. THF) can be used. Alcohols can also be used, however, their concentration must be higher to achieve the same degree of retention for an analyte relative to an aprotic solvent-water combination. Ionic additives, such as ammonium acetate and ammonium formate, are usually used to control the mobile phase pH and ion strength.

Samples may be prepared using methods known in the art and suitable for the extraction of lipids. In one embodiment, a modified Bligh-Dyer protocol is used as described below. To a certain volume/weight of the samples in a 13 X 100 mm glass screw capped tubes, H₂O is added to a final 1 mL volume. The sample is allowed to sit at room temperature for 10 min. 2 mL of MeOH and 0.9 mL CH₂Cl₂ are added to the sample and the sample is vortexed. If the sample is not monophasic, and if not add 50 µl MeOH to the sample and vortex (repeat as needed until the sample is monophasic). Add ULQS (i.e., internal standards), vortex, and incubate samples at room temperature for 30 min. Add 1 mL H₂O and 0.9 mL of CH₂Cl₂ to the samples and mix by inversion. Centrifuge the sample at 3400 rpm for 15 min. Collect the lower layer from the sample tube and transfer to a fresh glass tube. Add 2 mL CH₂Cl₂ to the sample remaining in the extraction tube and mix by inversion. Centrifuge the sample at 3400 rpm for 15 min. Collect the lower layer and add to the first extract. Evaporate solvent (for example, using a GeneVac) and resuspend the lipids in 50:50 mixture (vol:vol) of MeOH:CH₂Cl₂ and use or store at -20 °C until use. When used, the lipid samples are diluted in mobile phase A for LCMS analysis.

In this example, liquid chromatography (HILIC) is carried out using a Sciex ExionLC with AD Pump with a Luna 3 µm NH₂, 2X100 mm column (although any appropriate instrument and column may be used). Mobile Phase A and B are as follows: Mobile Phase A: 10:90 (vol:vol) CHCl₃:ACN + 1mM NH₄AC; Mobile Phase B: 50:50 (vol:vol) H₂O:ACN + 1mM NH₄AC. Typical injection volume is 5 µl and typical column temperature is 35 °C. In this example, a total run time of 14.00 min is used with the following gradient:

| Gradient | | | |
|---|---|---|---|
| Time (min) | Flow rate (ml/min) | Mobile Phase A (%) | Mobile Phase B (%) |
| 0.00 | 0.2 | 100 | 0 |
| 2.00 | 0.2 | 100 | 0 |
| 2.10 | 1.0 | 100 | 0 |
| 11.00 | 1.0 | 50 | 50 |
| 11.50 | 1.0 | 30 | 70 |
| 12.50 | 1.0 | 30 | 70 |
| 12.60 | 0.2 | 100 | 0 |
| 14.00 | 0.2 | 100 | 0 |

The liquid chromatography output is introduced directly into the mass spectrometer. In this example, MS is carried out using a Sciex 6500+ Triple Quad mass spectrometer (although any appropriate instrument and column may be used). Operating conditions are given in the table below.

| | Positive mode | Negative mode |
|---|---|---|
| Curtain gas | 20 psi | 20 psi |
| Collision gas | 8 | 8 |
| Ionspray voltage | 5500 | -4500 |
| Source Temperature | 150 °C | 150 °C |
| GS1 | 30 psi | 30 psi |
| GS2 | 30 psi | 30 psi |

MRM parameters are: MRM detection window- 30 sec; and target scan time- 0.3 sec.

### Example 2

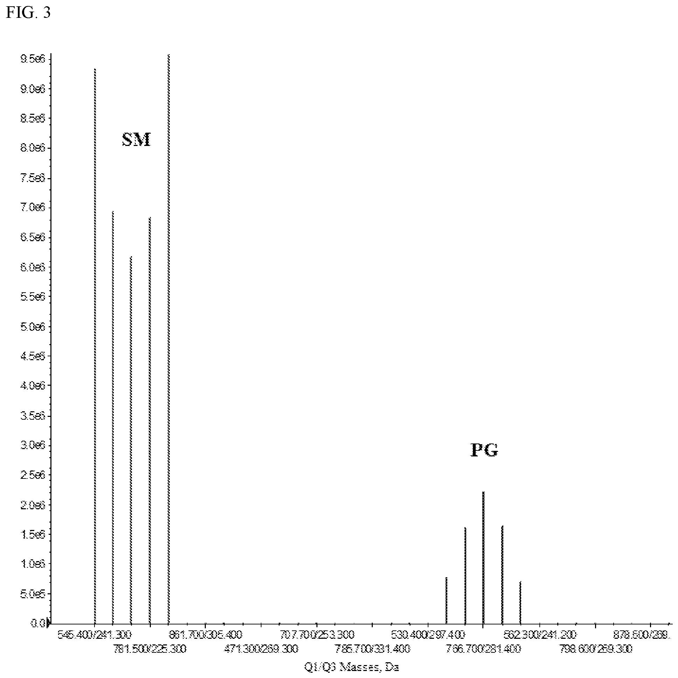

This Example show a representative distribution of ULQS internal standards for the SM and PG lipid using In this example, HILIC classes illustrating the concentration variations of the internal standards that are modeled after the typical distribution of acyl chain lengths and variation in saturation of naturally occurring lipids within SM and PG classes. FIG. 3 shows a difference in pattern abundance between the two the ULQS SM and PG classes for different species within each class. valley versus peak shaped. These spectra were obtained using HILIC based chromatography on a Sciex^{®} Exion LC system in conjunction with a Sciex^{®} 6500+ Triple Quadrupole mass spectrometer.

In this example, the MS procedure used was as described in Example 1 using liquid chromatography (HILIC), with the exception that Mobile Phase A was 10:90 (vol:vol) CHCl₃:ACN + 2mM NH₄AC; Mobile Phase B: 50:50 (vol:vol) H₂O:ACN + 2mM NH₄AC.

### Example 3

This Example illustrates the benefits of having multiple internal standards for one or more classes of lipids. The presence of multiple ULQS internal standards accounts for the variation in acyl chain length and saturation/unsaturation of acyl chains observed in naturally occurring lipid species in biological systems. These differences are known to affect the ability of lipids in a sample to ionize and fragment, thus producing a different signal response when using mass spectrometry techniques. The foregoing often results in quantitative error when only one internal standard is present in a sample and applied across multiple different naturally occurring species.

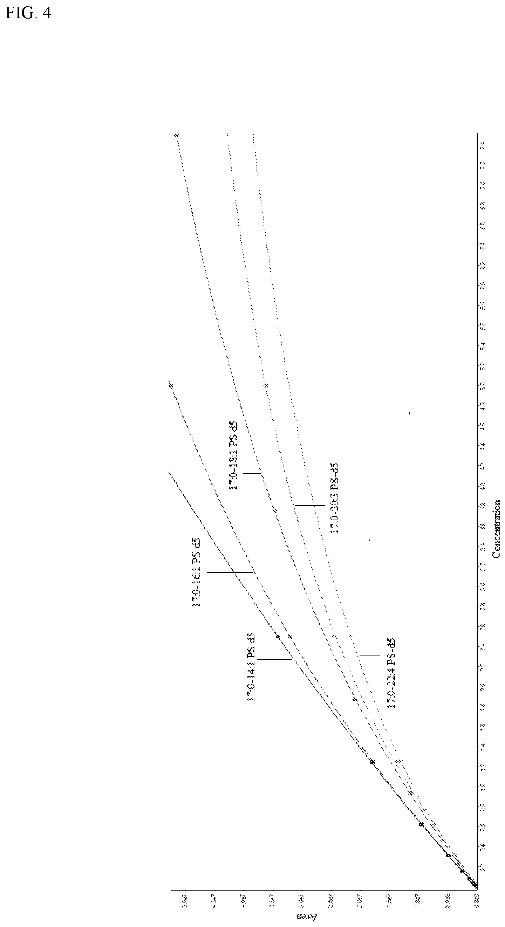

FIG. 4 shows a calibration curves generated using 5 ULQS PS internal standards. FIG. 4 illustrates a change in linearity for each ULQS PS internal standard species and illustrates the need for multiple internal standards to account for the variation in acyl chain lengths and saturation/unsaturation observed in naturally occurring lipid species. The use of the ULQS internal standards addresses the problem of variability observed in naturally occurring lipid species resulting from differences in ionization efficiency and differential fragmentation efficiency. These concentration curves were obtained using HILIC based chromatography on a Sciex^{®} Exion LC system in conjunction with a Sciex^{®} 6500+ Triple Quadrupole mass spectrometer.

In this example, the MS procedure used was as described in Example 1 using liquid chromatography (HILIC), with the exception that Mobile Phase A was 10:90 (vol:vol) CHCl₃:ACN + 2mM NH₄AC; Mobile Phase B: 50:50 (vol:vol) H₂O:ACN + 2mM NH₄AC.

## Claims

1. A universal lipid quantitative standard (ULQS) comprising a plurality of isotopically labeled lipid standards, wherein the plurality of isotopically labeled lipid standards includes
at least one lipid species from one or more lipid classes selected from the group consisting of: a lysophospholipid class, a cholesterol ester class, a triacylglycerol class, a diacylglycerol class, a ceramide class, and a sphingomyelin class, and,
at least one lipid species from the phospholipid class which includes one or more phospholipid species selected from the group consisting of: phosphatidylcholine (PC), phosphatidylethanolamine (PE), phosphatidylserine (PS), phosphatidylglycerol (PG), and phosphatidylinositol (PI).

2. The ULQS of claim 1, wherein the plurality of isotopically labeled lipid standards includes:
a. 15 phospholipid species, 10 lysophospholipid species, 3 cholesterol ester species, 5 triacylglycerol species, 3 diacylglycerol species, 3 ceramide species, and 3 sphingomyelin species
b. 20 phospholipid species, 15 lysophospholipid species, 5 cholesterol ester species, 9 triacylglycerol species, 5 diacylglycerol species, 5 ceramide species, and 5 sphingomyelin species; or
c. 25 phospholipid species, 15 lysophospholipid species, 5 cholesterol ester species, 9 triacylglycerol species, 5 diacylglycerol species, 5 ceramide species, and 5 sphingomyelin species.

3. The ULQS of claim 1 or 2, wherein the lipid species for each lipid class are selected to correct for at least one of the following: ionization efficiency, extraction efficiency, and differential fragmentation efficiency of the lipid species in a sample.

4. The ULQS of any preceding claim, wherein the plurality of isotopically labeled lipid standards includes at least one lipid species from the at least one lipid species from the phospholipid class as represented by the general formula I, or a pharmaceutically acceptable salt thereof: 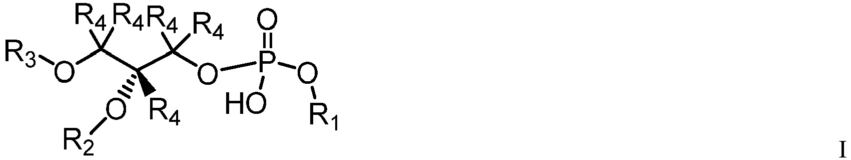 wherein:
R₁ is -(CH₂)ₙ-N(CH₃)₃, -(CH₂)ₙ-NH₃, -(CH₂)ₙ-C(H)(NH3)-C(O)-O⁻, -(CH₂)ₙ-CH(OH)-CH(OH), inositol, and H;
n is 1 to 6;
R₂ is a C3 to C30 saturated or unsaturated acyl chain;
R₃ is a C3 to C30 saturated or unsaturated acyl chain; and
R₄ is independently H or an isotope of H, provided that at least one of R₄ is an isotope of H.

5. The ULQS of any preceding claim , wherein the lysophospholipid class includes one or more lysophospholipid species selected from the group consisting of: lysophosphatidylcholine (LPC), lysophosphatidylethanolamine (LPE), lysophosphatidylsphoserine (LPS), lysophosphatidylglycerol (LPG), and lysophosphatidylinositol (LPI).

6. The ULQS of claim 5, wherein the plurality of isotopically labeled lipid standards includes at least one lipid species from the lysophospholipid class includes one or more the lysophospholipid species are represented by the general formula II, or a pharmaceutically acceptable salt thereof: 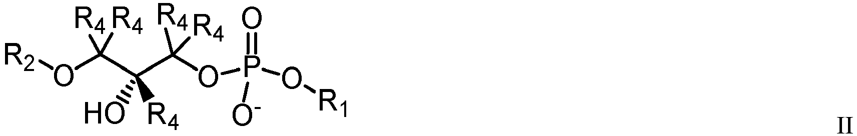 wherein:
R₁ is -(CH₂)ₙ-N(CH₃)₃, -(CH₂)ₙ-NH₃, -(CH₂)ₙ-C(H)(NH3)-C(O)-O⁻, -(CH₂)ₙ-CH(OH)-CH(OH), inositol, and H;
n is 1 to 6;
R₂ is a C2 to C24 saturated or unsaturated acyl chain; and
R₄ is independently H or an isotope of H, provided that at least one of R₄ is an isotope of H.

7. The ULQS of any preceding claim , wherein the plurality of isotopically labeled lipid standards includes at least one lipid species selected from a cholesterol ester species and wherein the cholesterol ester species are represented by the general formula III, or a pharmaceutically acceptable salt thereof: 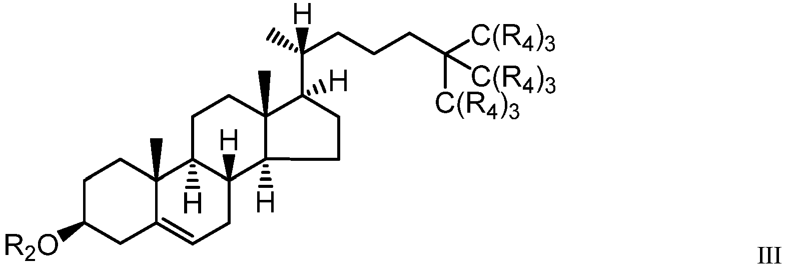 wherein:
R₂ is a C9 to C29 saturated or unsaturated acyl chain; and
R₄ is independently H or an isotope of H, provided that at least one of R₄ is an isotope of H.

8. The ULQS of claim 1, wherein the plurality of isotopically labeled lipid standards includes at least one lipid species from the triacylglycerol class and wherein the triacylglycerol class includes one or more triacylglycerol species, and wherein the triacylglycerol species are represented by the general formula IV, or a pharmaceutically acceptable salt thereof: 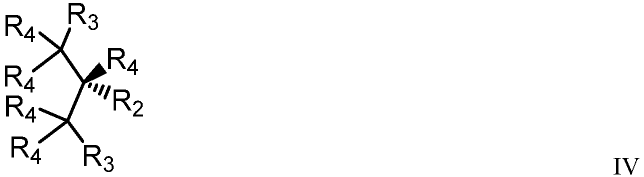 wherein:
R₂ is a C3 to C30 saturated or unsaturated acyl chain;
R₃ are each independently a C3 to C25 saturated or unsaturated acyl chain; and
R₄ is independently H or an isotope of H, provided that at least one of R₄ is an isotope of H.

9. The ULQS of any preceding claim , wherein the plurality of isotopically labeled lipid standards includes at least one lipid species from the diacylglycerol class and wherein the diacylglycerol class includes one or more diacylglycerol species, wherein the diacylglycerol species are represented by the general formula V, or a pharmaceutically acceptable salt thereof: 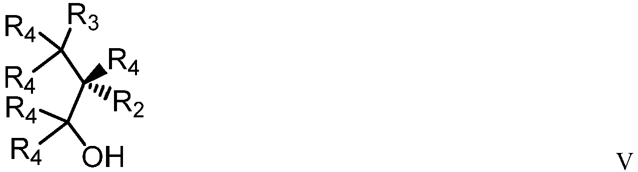 wherein:
R₂ is a C3 to C30 saturated or unsaturated acyl chain;
R₃ is a C3 to C25 saturated or unsaturated acyl chain; and
R₄ is independently H or an isotope of H, provided that at least one of R₄ is an isotope of H.

10. The ULQS of any preceding claim , wherein the plurality of isotopically labeled lipid standards includes at least one lipid species from the ceramide class and wherein the ceramide class includes one or more ceramide species, wherein the ceramide species are represented by the general formula VI, or a pharmaceutically acceptable salt thereof: 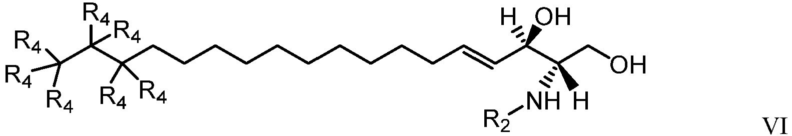 wherein:
R₂ is a C10 to C30 saturated or unsaturated acyl chain; and
R₄ is independently H or an isotope of H, provided that at least one of R₄ is an isotope of
H.

11. The ULQS of any preceding claim , wherein the plurality of isotopically labeled lipid standards includes at least one lipid species from the sphingomyelin class and wherein the sphingomyelin class includes one or more sphingomyelin species, wherein the sphingomyelin species are represented by the general formula VII, or a pharmaceutically acceptable salt thereof: 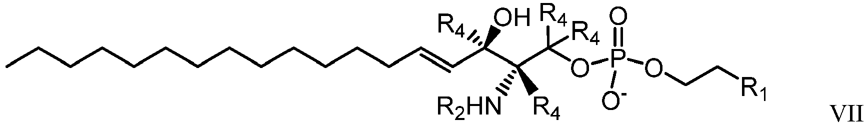 wherein:
R₁ is 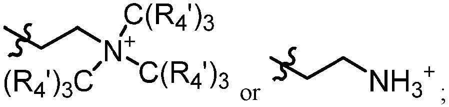
R₂ is a C10 to C30 saturated or unsaturated acyl chain; and
R₄ and R₄' are each independently H or an isotope of H, provided that at least one of R₄ or R₄' is an isotope of H.

12. The ULQS of claim 11, wherein R₁ is 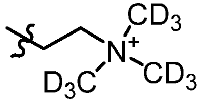 and R₄ is H.

13. The ULQS of claim 11, wherein R₁ is 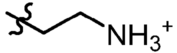 and at least one of R₄ is an isotope of H.

14. The ULQS of any preceeding claim, comprising at least one lipid species from each of the lysophospholipid class, the cholesterol ester class, the triacylglycerol class, the diacylglycerol class, the ceramide class, and the sphingomyelin class.

15. The ULQS of claim 1 comprising 5 to 40 phospholipid species, 5 to 25 lysophospholipid species, 3 to 15 cholesterol ester species, 5 to 20 triacylglycerol species, 3 to 15 diacylglycerol species, 3 to 15 ceramide species, and 3 to 15 sphingomyelin species.
